# EUROPEAN PATENT SPECIFICATION

(11) **EP 4 356 842 B1**
(45) Date of publication and mention of the grant of the patent: **02.07.2025**
(21) Application number: 23204213.5
(22) Date of filing: 17.10.2023
(51) Int. Cl.: A61B 8/08

(54) **ULTRASOUND DIAGNOSTIC APPARATUS AND CONTROL METHOD OF ULTRASOUND DIAGNOSTIC APPARATUS**
ULTRASCHALLDIAGNOSEVORRICHTUNG UND STEUERUNGSVERFAHREN FÜR DIE ULTRASCHALLDIAGNOSEVORRICHTUNG
APPAREIL DE DIAGNOSTIC À ULTRASONS ET PROCÉDÉ DE COMMANDE D'APPAREIL DE DIAGNOSTIC À ULTRASONS

(30) Priority: 17.10.2022 JP 2022166347
(43) Date of publication of application: 24.04.2024
(73) Proprietor: FUJI-FILM Corporation, Minato-ku Tokyo 106-8620 (JP)
(72) Inventor: MATSUMOTO, Tsuyoshi, Kanagawa, 258-8538 (JP); INOUE, Tomoki, Kanagawa, 258-8538 (JP)
(74) Representative: Dehns Germany Partnerschaft mbB

(56) References cited:
- US-A1- 2008 269 605
- US-A1- 2021 128 099

## Description

### BACKGROUND OF THE INVENTION

### 1. Field of the Invention

The present invention relates to an ultrasound diagnostic apparatus and a control method of an ultrasound diagnostic apparatus for examining a blood vessel of a subject.

### 2. Description of the Related Art

Conventionally, a technique is known for using a so-called ultrasound diagnostic apparatus to capture an ultrasound image of a blood vessel of a subject while compressing a body surface of the subject with an ultrasound probe, for example, in an examination for checking the presence or absence of so-called lower limb varicose veins, an examination for so-called deep vein thrombosis, and the like. In order to appropriately perform the examination performed while compressing the body surface of the subject with the ultrasound probe, a user of the ultrasound diagnostic apparatus requires a certain proficiency level or higher in increasing and reducing a compression force applied by the ultrasound probe and the like. In that respect, in order to facilitate such an examination, for example, technologies disclosed in JP2016-123794A, JP2016-042903A, JP2020-512069A, and JP2006-263128Ahave been developed.

JP2016-123794A discloses a technology for determining whether or not a compression state is proper by detecting a compression force applied by an ultrasound probe with a sensor. JP2016-042903A discloses a technology for measuring blood pressure using a compression force applied by an ultrasound probe in a case where a degree of deformation of a blood vessel inside a subject reaches a certain value. JP2020-512069A discloses a technology for estimating a pressure on a body surface of a subject by an ultrasound probe by analyzing an ultrasound image using a so-called machine learning method, and then determining whether or not a blood vessel is narrowed in a case where a certain pressure is applied to the body surface of the subject. JP2006-263128A discloses a technology for calculating an elastic modulus of a blood vessel based on a compression force in a case where a body surface of a subject is compressed using an ultrasound probe.

### SUMMARY OF THE INVENTION

Meanwhile, it is known that in an examination performed while compressing a body surface of a subject with an ultrasound probe, there is an appropriate compression force applied by the ultrasound probe depending on a site of the subject. In particular, a user having a low proficiency level may not be able to compress the body surface of the subject with a compression force suitable for an examination site even in a case where the technologies disclosed in JP2016-123794A, JP2016-042903A, JP2020-512069A, and JP2006-263128A are used. In addition, in a case where the ultrasound diagnostic apparatus has a plurality of examination modes, for example, it is conceivable to select an examination mode suitable for the examination site and then use the technologies disclosed in JP2016-123794A, JP2016-042903A, JP2020-512069A, and JP2006-263128A. However, there may be difficulties in smoothly performing the examination, for example, due to an unintended movement of a hand holding the ultrasound probe when sequentially switching the examination modes during the examination, which hinders the capturing of the ultrasound image.

The present invention has been made in order to solve such a conventional problem, and an object of the present invention is to provide an ultrasound diagnostic apparatus and a control method of an ultrasound diagnostic apparatus that enable a user to smoothly and appropriately perform an examination.

According to the following configuration, the above-described object can be achieved.
[1] An ultrasound diagnostic apparatus that examines a blood vessel of a subject, the ultrasound diagnostic apparatus comprising:
   an ultrasound probe;
   an image acquisition unit configured to continuously acquire an ultrasound image of the blood vessel using the ultrasound probe;
   a blood vessel detection unit configured to detect the blood vessel from the ultrasound image;
   an examination site discrimination unit configured to discriminate an examination site;
   an examination mode setting unit configured to set an examination mode corresponding to the examination site discriminated by the examination site discrimination unit;
   a compression detection unit configured to detect a compression motion on a body surface of the subject by the ultrasound probe;
   a compression motion determination unit configured to determine whether or not the compression motion detected by the compression detection unit is proper for the examination mode set by the examination mode setting unit; and
   a notification unit configured to notify a user of a determination result by the compression motion determination unit.
[2] The ultrasound diagnostic apparatus according to [1],
   in which the examination site discrimination unit is configured to discriminate the examination site based on the ultrasound image.
[3] The ultrasound diagnostic apparatus according to [1] or [2], further comprising:
   an optical camera configured to image the subject and the ultrasound probe to acquire an optical image,
   in which the examination site discrimination unit is configured to discriminate the examination site based on the optical image.
[4] The ultrasound diagnostic apparatus according to any one of [1] to [3],
   in which the examination mode setting unit is configured to set a vein search mode in a case where the examination site is an upper limb.
[5] The ultrasound diagnostic apparatus according to any one of [1] to [4],
   in which the examination mode setting unit is configured to set a thrombosis determination mode in a case where the examination site is a lower limb.
[6] The ultrasound diagnostic apparatus according to any one of [1] to [5],
   in which the examination mode setting unit is configured to set an elastic modulus measurement mode in a case where the examination site is a neck.
[7] The ultrasound diagnostic apparatus according to any one of [1] to [6],
   in which the compression detection unit is configured to detect the compression motion based on a change in a diameter in a depth direction of the blood vessel in the ultrasound image.
[8] The ultrasound diagnostic apparatus according to [1] or [2], further comprising:
   an optical camera configured to image the subject and the ultrasound probe to acquire an optical image,
   in which the compression detection unit is configured to detect the compression motion based on a degree of depression of the body surface of the subject in the optical image or a movement of an arm of the user.
[9] The ultrasound diagnostic apparatus according to any one of [1] to [6], further comprising:
   a projection mapping device configured to project a moire pattern onto the body surface of the subject,
   in which the compression detection unit is configured to detect the compression motion based on a change in the moire pattern projected onto the body surface of the subject.
[10] The ultrasound diagnostic apparatus according to any one of [1] to [6], further comprising:
   a pressure sensor or an acceleration sensor attached to the ultrasound probe,
   in which the compression detection unit is configured to detect the compression motion based on a change in an output value of the pressure sensor or the acceleration sensor.
[11] The ultrasound diagnostic apparatus according to any one of [1] to [10],
   in which the compression motion determination unit has a predetermined allowable range of the compression motion for each examination mode and is configured to determine whether or not the compression motion is proper by comparing the compression motion detected by the compression detection unit with the allowable range corresponding to the examination mode set by the examination mode setting unit.
[12] The ultrasound diagnostic apparatus according to [11], further comprising:
   a subject specification unit configured to specify the subject in response to an input of identification information of the subject,
   in which the compression motion determination unit is configured to adjust the allowable range according to age and sex of the subject specified by the subject specification unit.
[13] The ultrasound diagnostic apparatus according to [11], further comprising:
   a muscle mass recognition unit configured to recognize a muscle mass of the subject from the ultrasound image,
   in which the compression motion determination unit is configured to adjust the allowable range according to the muscle mass recognized by the muscle mass recognition unit.
[14] The ultrasound diagnostic apparatus according to [11], further comprising:
   an optical camera configured to image the subject to acquire an optical image; and
   an examination site thickness recognition unit configured to recognize a thickness of the examination site from the optical image acquired by the optical camera,
   in which the compression motion determination unit is configured to adjust the allowable range according to the thickness of the examination site recognized by the examination site thickness recognition unit.
[15] The ultrasound diagnostic apparatus according to any one of [11] to [14], further comprising:
   a compression force adjustment recommendation unit configured to, in a case where the compression motion is determined to be improper by the compression motion determination unit, recommend adjusting a compression force of the ultrasound probe on the body surface of the subject such that the compression motion falls within the allowable range,
   in which the notification unit is configured to notify the user of the adjustment of the compression force recommended by the compression force adjustment recommendation unit.
[16] The ultrasound diagnostic apparatus according to any one of [1] to [15], further comprising:
   a proficiency level discrimination unit configured to discriminate a proficiency level of the user; and
   a determination operation stop unit configured to stop an operation of determination by the compression motion determination unit according to the proficiency level of the user discriminated by the proficiency level discrimination unit.
[17] The ultrasound diagnostic apparatus according to any one of [1] to [16], further comprising:
   a user specification unit configured to specify the user in response to an input of identification information of the user,
   in which the notification unit is configured to notify the user of a message corresponding to the user specified by the user specification unit.
[18] A control method of an ultrasound diagnostic apparatus that examines a blood vessel of a subject, the control method comprising:
   continuously acquiring an ultrasound image of the blood vessel using an ultrasound probe;
   detecting the blood vessel from the ultrasound image;
   discriminating an examination site;
   setting an examination mode corresponding to the discriminated examination site;
   detecting a compression motion on a body surface of the subject by the ultrasound probe;
   determining whether or not the compression motion detected for the set examination mode is proper; and
   notifying a user of a determination result.

According to the present invention, there is provided an ultrasound diagnostic apparatus that examines a blood vessel of a subject, the ultrasound diagnostic apparatus comprising: an ultrasound probe; an image acquisition unit that continuously acquires an ultrasound image of the blood vessel using the ultrasound probe; a blood vessel detection unit that detects the blood vessel from the ultrasound image; an examination site discrimination unit that discriminates an examination site; an examination mode setting unit that sets an examination mode corresponding to the examination site discriminated by the examination site discrimination unit; a compression detection unit that detects a compression motion on a body surface of the subject by the ultrasound probe; a compression motion determination unit that determines whether or not the compression motion detected by the compression detection unit is proper for the examination mode set by the examination mode setting unit; and a notification unit that notifies a user of a determination result by the compression motion determination unit. Therefore, the user can smoothly and appropriately perform an examination.

### BRIEF DESCRIPTION OF THE DRAWINGS

Fig. 1 is a block diagram showing a configuration of an ultrasound diagnostic apparatus according to Embodiment 1 of the present invention.
Fig. 2 is a block diagram showing a configuration of a transmission and reception circuit in Embodiment 1 of the present invention.
Fig. 3 is a block diagram showing a configuration of an image generation unit in Embodiment 1 of the present invention.
Fig. 4 is a diagram showing an example of an ultrasound image of a blood vessel imaged in a state in which a body surface of a subject is not compressed by the ultrasound probe.
Fig. 5 is a diagram showing an example of an ultrasound image of the blood vessel imaged in a state in which the body surface of the subject is compressed by the ultrasound probe.
Fig. 6 is a flowchart showing an operation of the ultrasound diagnostic apparatus according to Embodiment 1 of the present invention.
Fig. 7 is a block diagram showing a configuration of an ultrasound diagnostic apparatus according to Embodiment 2 of the present invention.
Fig. 8 is a block diagram showing a configuration of an ultrasound diagnostic apparatus according to Embodiment 3 of the present invention.
Fig. 9 is a block diagram showing a configuration of an ultrasound diagnostic apparatus according to Embodiment 4 of the present invention.
Fig. 10 is a block diagram showing a configuration of an ultrasound diagnostic apparatus according to Embodiment 5 of the present invention.
Fig. 11 is a block diagram showing a configuration of an ultrasound diagnostic apparatus according to Embodiment 6 of the present invention.
Fig. 12 is a block diagram showing a configuration of an ultrasound diagnostic apparatus according to Embodiment 7 of the present invention.
Fig. 13 is a block diagram showing a configuration of an ultrasound diagnostic apparatus according to Embodiment 8 of the present invention.

### DESCRIPTION OF THE PREFERRED EMBODIMENTS

Hereinafter, embodiments of the present invention will be described with reference to the accompanying drawings.

The description of configuration requirements to be described below is made based on a representative embodiment of the present invention, but the present invention is not limited to such an embodiment.

In the present specification, a numerical range represented by "to" means a range including numerical values described before and after "to" as a lower limit value and an upper limit value, respectively.

In the present specification, "same" and "identical" include an error range generally allowed in the technical field.

### Embodiment 1

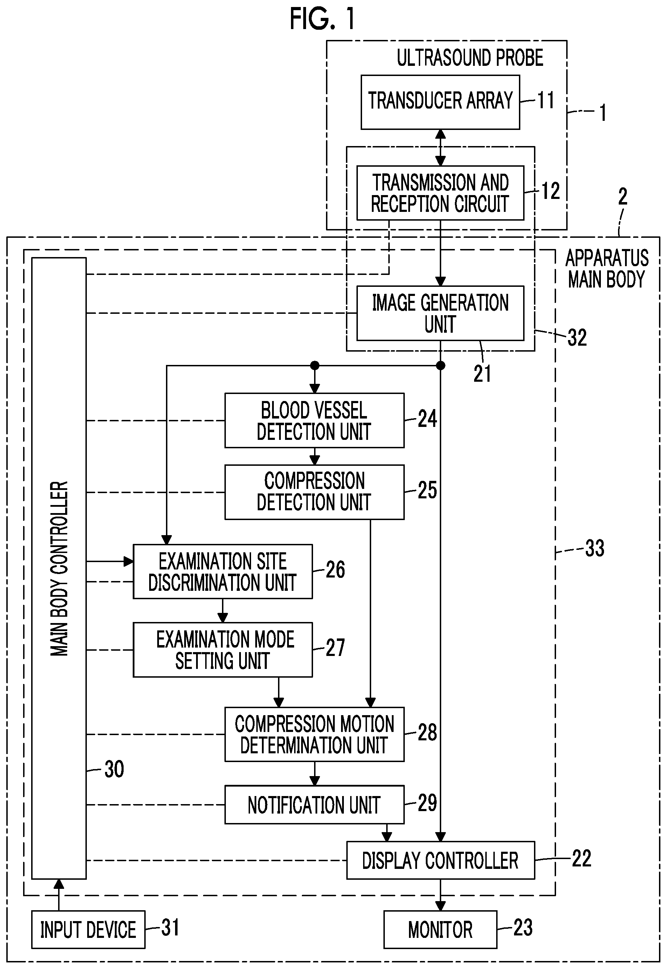

Fig. 1 shows a configuration of an ultrasound diagnostic apparatus according to Embodiment 1 of the present invention. The ultrasound diagnostic apparatus comprises an ultrasound probe 1 and an apparatus main body 2 connected to the ultrasound probe 1. The ultrasound diagnostic apparatus of the embodiment of the present invention is used to examine a blood vessel of a subject while compressing a body surface of the subject with the ultrasound probe 1.

The ultrasound probe 1 includes a transducer array 11. A transmission and reception circuit 12 is connected to the transducer array 11.

The apparatus main body 2 includes an image generation unit 21 connected to the transmission and reception circuit 12 of the ultrasound probe 1. A display controller 22 and a monitor 23 are sequentially connected to the image generation unit 21. In addition, a blood vessel detection unit 24 is connected to the image generation unit 21. A compression detection unit 25 is connected to the blood vessel detection unit 24. Further, an examination site discrimination unit 26 is connected to the image generation unit 21. An examination mode setting unit 27 is connected to the examination site discrimination unit 26. Further, a compression motion determination unit 28 is connected to the compression detection unit 25 and the examination mode setting unit 27. A notification unit 29 is connected to the compression motion determination unit 28. The notification unit 29 is connected to the display controller 22. Further, a main body controller 30 is connected to the image generation unit 21, the display controller 22, the blood vessel detection unit 24, the compression detection unit 25, the examination site discrimination unit 26, the examination mode setting unit 27, the compression motion determination unit 28, and the notification unit 29. An input device 31 is connected to the main body controller 30.

In addition, the transmission and reception circuit 12 and the image generation unit 21 constitute an image acquisition unit 32. Further, the image generation unit 21, the display controller 22, the blood vessel detection unit 24, the compression detection unit 25, the examination site discrimination unit 26, the examination mode setting unit 27, the compression motion determination unit 28, the notification unit 29, and the main body controller 30 constitute a processor 33 for the apparatus main body 2.

The transducer array 11 of the ultrasound probe 1 includes a plurality of ultrasound transducers one-dimensionally or two-dimensionally arranged. Each of these ultrasound transducers transmits an ultrasound wave in accordance with a drive signal supplied from the transmission and reception circuit 12 and receives an ultrasound echo from a subject to output a signal based on the ultrasound echo. For example, each ultrasound transducer is composed of a piezoelectric body consisting of a piezoelectric ceramic represented by lead zirconate titanate (PZT), a polymer piezoelectric element represented by poly vinylidene di fluoride (PVDF), a piezoelectric single crystal represented by lead magnesium niobate-lead titanate (PMN-PT), or the like, and electrodes formed at both ends of the piezoelectric body.

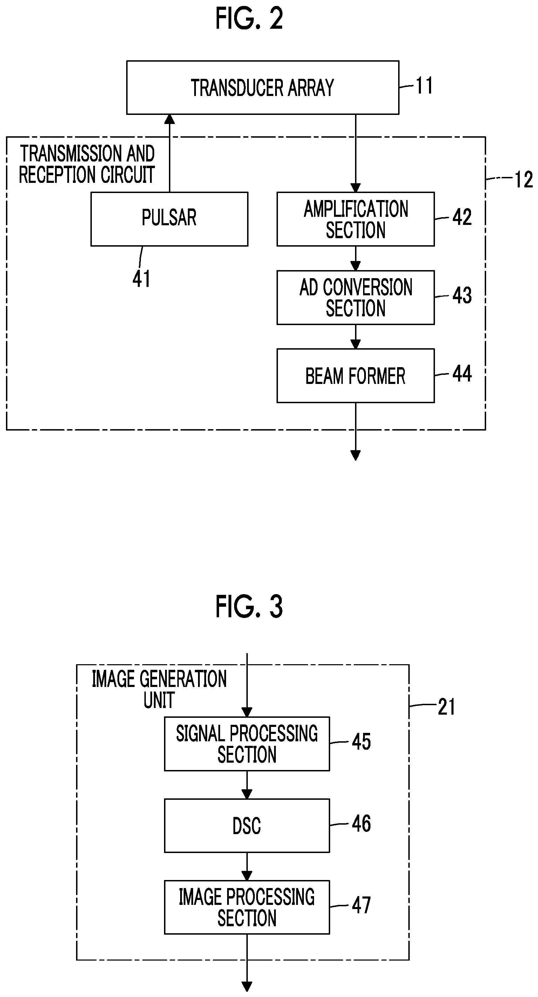

The transmission and reception circuit 12 transmits the ultrasound wave from the transducer array 11 and generates a sound ray signal based on a reception signal acquired by the transducer array 11, under the control of the main body controller 30. As shown in Fig. 2, the transmission and reception circuit 12 includes a pulsar 41 connected to the transducer array 11, and an amplification section 42, an analog-to-digital (AD) conversion section 43, and a beam former 44 that are sequentially connected in series to the transducer array 11.

The pulsar 41 includes, for example, a plurality of pulse generators, and adjusts an amount of delay of each of drive signals and supplies the drive signals to the plurality of ultrasound transducers such that ultrasound waves transmitted from the plurality of ultrasound transducers of the transducer array 11 form an ultrasound beam based on a transmission delay pattern selected according to a control signal from the main body controller 30. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, whereby an ultrasound beam is formed from the combined wave of these ultrasound waves.

The transmitted ultrasound beam is reflected in, for example, a target such as a site of the subject and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate a reception signal, which is an electrical signal, and outputs these reception signals to the amplification section 42.

The amplification section 42 amplifies the signal input from each of the ultrasound transducers constituting the transducer array 11 and transmits the amplified signal to the AD conversion section 43. The AD conversion section 43 converts the signal transmitted from the amplification section 42 into digital reception data. The beam former 44 performs so-called reception focus processing by applying and adding a delay to each reception data received from the AD conversion section 43. By this reception focus processing, each reception data converted by the AD conversion section 43 is phase-added, and a sound ray signal in which the focus of the ultrasound echo is narrowed down is acquired.

As shown in Fig. 3, the image generation unit 21 has a configuration in which a signal processing section 45, a digital scan converter (DSC) 46, and an image processing section 47 are sequentially connected in series.

The signal processing section 45 generates a B-mode image signal, which is tomographic image information regarding tissues inside the subject, by performing, on the sound ray signal received from the transmission and reception circuit 12, correction of the attenuation due to the distance according to the depth of the reflection position of the ultrasound wave using a sound velocity value set by the main body controller 30 and then performing envelope detection processing.

The DSC 46 converts (raster-converts) the B-mode image signal generated by the signal processing section 45 into an image signal in accordance with a normal television signal scanning method.

The image processing section 47 performs various types of necessary image processing such as gradation processing on the B-mode image signal input from the DSC 46 and then sends out the B-mode image signal to the display controller 22, the blood vessel detection unit 24, and the examination site discrimination unit 26. Hereinafter, the B-mode image signal that has been subjected to image processing by the image processing section 47 is referred to as an ultrasound image.

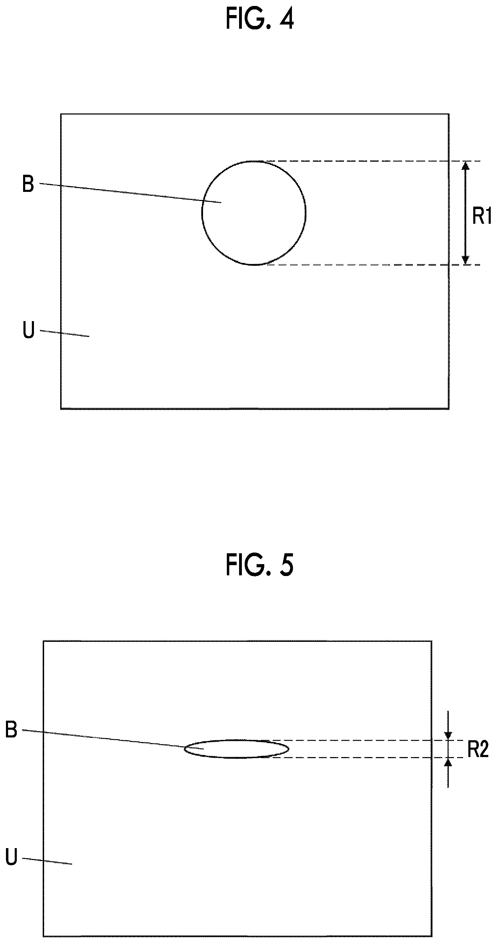

In the present invention, for example, as shown in Fig. 4, an ultrasound image U showing a blood vessel B inside the subject is acquired. Hereinafter, unless otherwise specified, the ultrasound image U showing a short-axis image of the blood vessel B is simply referred to as the ultrasound image U showing the blood vessel B. The short-axis image of the blood vessel B refers to a cross section of the blood vessel B perpendicular to a running direction of the blood vessel B.

The display controller 22 performs predetermined processing on the ultrasound image or the like generated by the image generation unit 21 and displays the ultrasound image or the like on the monitor 23, under the control of the main body controller 30.

The monitor 23 performs various kinds of display under the control of the display controller 22. The monitor 23 can include, for example, a display device such as a liquid crystal display (LCD) or an organic electroluminescence (EL) display.

The main body controller 30 controls each unit of the apparatus main body 2 and the ultrasound probe 1 in accordance with a program recorded in advance, or the like.

The input device 31 accepts an input operation from an examiner and sends out input information to the main body controller 30. The input device 31 is composed of, for example, a device for the examiner to perform an input operation, such as a keyboard, a mouse, a trackball, a touchpad, or a touch panel.

The blood vessel detection unit 24 detects the blood vessel B shown in the ultrasound image U by analyzing the ultrasound image U. The blood vessel detection unit 24 stores a plurality of template images related to the short-axis image of the blood vessel B, and can detect the blood vessel B using a so-called template matching method of searching the ultrasound image U using the plurality of template images. The blood vessel detection unit 24 can also detect the blood vessel B from the ultrasound image U using, for example, a trained model in so-called machine learning, which has been trained using a large number of ultrasound images U showing the short-axis image of the blood vessel B.

The compression detection unit 25 detects a compression motion on the body surface of the subject by the ultrasound probe 1. Here, in a case where the body surface of the subject is compressed by the ultrasound probe 1, the blood vessel B, particularly the vein, in the subject changes from a shape shown in Fig. 4 to a compressed shape in a depth direction as shown in Fig. 5. In that respect, the compression detection unit 25 can detect the compression motion based on, for example, a change in the diameter in the depth direction of the blood vessel B detected by the blood vessel detection unit 24.

In this case, the compression detection unit 25 can calculate, for example, a ratio R2/R1 between a diameter R1 in the depth direction of the blood vessel B in a state in which no compression is applied by the ultrasound probe 1 and a diameter R2 in the depth direction of the blood vessel B in a state in which compression is applied by the ultrasound probe 1 to detect the compression motion in a case where the ratio R2/R1 is equal to or less than a predetermined ratio threshold value. In addition, the compression detection unit 25 can send out, for example, the ratio R2/R1 to the compression motion determination unit 28 as a compression motion indicator representing the magnitude of the compression motion by the ultrasound probe 1.

The examination site discrimination unit 26 analyzes the ultrasound image U generated by the image generation unit 21 to discriminate an examination site captured in the ultrasound image U, such as an upper limb, a lower limb, or a neck, for example. The examination site discrimination unit 26 can detect, for example, a target such as the vein, the artery, the bone, and the muscle shown in the ultrasound image U to discriminate the examination site based on the disposition position and the size of the detected target. In this case, the examination site discrimination unit 26 stores a plurality of template images showing the target, such as the vein, the artery, the bone, and the muscle, and can detect the disposition position and the size of the target using a so-called template matching method of searching the inside of the ultrasound image U using these template images. The examination site discrimination unit 26 can further store in advance a plurality of combinations of the disposition position and the size of the target such as the vein, the artery, the bone, and the muscle, and the examination site to discriminate the examination site by fitting the disposition position and the size of the detected target to the stored combination.

In addition, the examination site discrimination unit 26 can also perform the detection of the target such as the vein, the artery, the bone, and the muscle, and the discrimination of the examination site by using a trained model in so-called machine learning, which has learned in advance a large number of ultrasound images U showing the target such as the vein, the artery, the bone, and the muscle, and a large number of combinations of the disposition position and the size of the target such as the vein, the artery, the bone, and the muscle, and the examination site.

In addition, the examination site discrimination unit 26 can analyze, for example, a plurality of continuous frames of ultrasound images U generated by the image generation unit 21 to detect organs shown in the plurality of frames of ultrasound images U, thereby discriminating the examination site based on the movement of the detected organs. In this case as well, the examination site discrimination unit 26 can discriminate the examination site using template matching or a trained model in machine learning.

Further, the examination site discrimination unit 26 can also discriminate the examination site based on, for example, text information, a so-called schema image, or the like input from a user via the input device 31.

Further, in general, in a case where the subject is examined using the ultrasound diagnostic apparatus, a plurality of predetermined examination sites may be examined according to predetermined procedures in accordance with predetermined examination protocols. In such a case, the examination site discrimination unit 26 can also sequentially recognize the types of sites of the subject imaged in the ultrasound image U to discriminate the examination site based on the order of the recognized sites.

The examination mode setting unit 27 sets an examination mode corresponding to the examination site discriminated by the examination site discrimination unit 26. Here, the examination mode corresponding to the examination site is, for example, a mode having imaging conditions such as a gain and a depth corresponding to the examination site in order to enable clear observation of the examination site.

For example, the examination mode setting unit 27 can set a vein search mode as the examination mode in a case where the examination site is discriminated to be the upper limb by the examination site discrimination unit 26. In addition, the examination mode setting unit 27 can set, for example, a thrombosis determination mode as the examination mode in a case where the examination site is discriminated to be the lower limb by the examination site discrimination unit 26. Further, the examination mode setting unit 27 can set an elastic modulus measurement mode as the examination mode in a case where the examination site is discriminated to be the neck by the examination site discrimination unit 26.

Further, since an appropriate compression motion by the ultrasound probe 1 differs for each examination site such as the upper limb, the lower limb, and the neck, criteria for determining whether or not the compression motion is proper differ depending on the examination mode, as will be described below.

The compression motion determination unit 28 determines whether or not the compression motion detected by the compression detection unit 25 is proper for the examination mode set by the examination mode setting unit 27. The compression motion determination unit 28 has a predetermined allowable range of the compression motion for each examination mode, and determines whether or not the compression motion is proper by comparing the compression motion detected by the compression detection unit 25 with the allowable range corresponding to the examination mode set by the examination mode setting unit 27. In this case, for example, the compression motion determination unit 28 receives the compression motion indicator representing the magnitude of the compression motion by the ultrasound probe 1 from the compression detection unit 25 to have a proper range of the compression motion indicator as the allowable range of the compression motion for each examination mode, and determines whether or not the compression motion is proper by comparing the compression motion indicator with the proper range thereof for the set examination mode.

Here, in general, the vein in the upper limb is often disposed at an extremely shallow position and thin. Therefore, in a case where the body surface of the subject is compressed by the ultrasound probe 1, the vein is significantly compressed in the depth direction, which may make it difficult to confirm the vein on the ultrasound image U. In addition, the blood vessel B in the neck is often disposed at a shallow position just beneath the epidermis. Therefore, for example, in a case of measuring the elastic modulus of the blood vessel B, it is desirable to apply a weak compression force using the ultrasound probe 1. Further, the blood vessel B in the lower limb is often disposed at a deep position where muscles and fats are present. Therefore, for example, in a case of examining whether or not thrombosis has occurred in the blood vessel B, it is desirable to apply a strong compression force using the ultrasound probe 1.

Therefore, for example, the compression motion determination unit 28 can set the allowable range of the compression motion in the vein search mode of the upper limb to be very small, can set the allowable range of the compression motion in the elastic modulus measurement mode of the neck to be larger than the allowable range of the compression motion in the vein search mode of the upper limb, and can set the allowable range of the compression motion in the thrombosis determination mode of the lower limb to be larger than the allowable range of the compression motion in the elastic modulus measurement mode of the neck.

As a specific example, in a case where the ratio R2/R1 is used as the compression motion indicator, the compression motion determination unit 28 can set the allowable range of the compression motion indicator in the vein search mode of the upper limb to a range of values in the vicinity of 1, can set the allowable range of the compression motion indicator in the elastic modulus measurement mode of the neck to a range smaller than the range of values in the vicinity of 1, and can set the allowable range of the compression motion indicator in the thrombosis determination mode of the lower limb to an even smaller range.

The notification unit 29 notifies the user of the determination result by the compression motion determination unit 28. In this case, the notification unit 29 can notify the user, for example, by displaying the determination result by the compression motion determination unit 28 on the monitor 23.

The user can easily perform the compression motion suitable for the examination site by applying the compression to the body surface of the subject using the ultrasound probe 1 while confirming the determination result obtained through the notification from the notification unit 29. In addition, since the examination mode is automatically set for the examination site automatically discriminated by the examination site discrimination unit 26, the user can smoothly and appropriately perform the examination without the need to, for example, manually switch the examination modes during the examination.

Although the processor 33 including the image generation unit 21, the display controller 22, the blood vessel detection unit 24, the compression detection unit 25, the examination site discrimination unit 26, the examination mode setting unit 27, the compression motion determination unit 28, the notification unit 29, and the main body controller 30 is composed of a central processing unit (CPU) and a control program for causing the CPU to perform various types of processing, the processor 33 may be composed of a field programmable gate array (FPGA), a digital signal processor (DSP), an application specific integrated circuit (ASIC), a graphics processing unit (GPU), or other integrated circuits (ICs), or may be composed of a combination thereof.

In addition, the image generation unit 21, the display controller 22, the blood vessel detection unit 24, the compression detection unit 25, the examination site discrimination unit 26, the examination mode setting unit 27, the compression motion determination unit 28, the notification unit 29, and the main body controller 30 of the processor 33 can also be configured by being integrated partially or entirely into one CPU or the like.

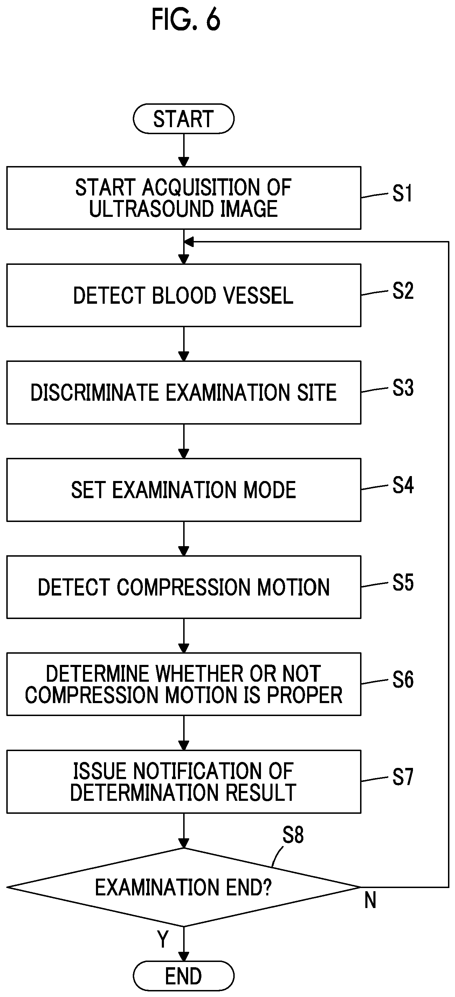

Next, an example of the operation of the ultrasound diagnostic apparatus according to Embodiment 1 will be described using the flowchart of Fig. 6.

First, in step S1, continuous acquisition of the ultrasound images U showing the blood vessel B of the subject in a state in which the user disposes the ultrasound probe 1 on the body surface of the subject is started. In this case, the transducer array 11 of the ultrasound probe 1 transmits the ultrasound beam into the subject and receives the ultrasound echo from the inside of the subject, thereby generating the reception signal. The transmission and reception circuit 12 of the image acquisition unit 32 performs so-called reception focus processing on the reception signal to generate the sound ray signal, under the control of the main body controller 30. The sound ray signal generated by the transmission and reception circuit 12 is sent out to the image generation unit 21. The image generation unit 21 generates the ultrasound image U using the sound ray signal sent out from the transmission and reception circuit 12.

Next, in step S2, the blood vessel detection unit 24 analyzes the ultrasound image U, for which the acquisition has been started in step S1, to detect the blood vessel B shown in the ultrasound image U. In this case, the blood vessel detection unit 24 can detect the blood vessel B using, for example, a template matching method and can also detect the blood vessel B using a trained model that has been trained using a large number of ultrasound images U showing the blood vessel B.

In step S3, the examination site discrimination unit 26 analyzes the ultrasound image U, for which acquisition is started in step S1, to discriminate the examination site captured in the ultrasound image U, such as the upper limb, the lower limb, or the neck, for example.

In this case, the examination site discrimination unit 26 can detect, for example, a target such as the vein, the artery, the bone, and the muscle shown in the ultrasound image U to discriminate the examination site based on the disposition position and the size of the detected target. In addition, the examination site discrimination unit 26 can also analyze, for example, a plurality of continuous frames of ultrasound images U generated by the image generation unit 21 to detect organs shown in the plurality of frames of ultrasound images U, thereby discriminating the examination site based on the movement of the detected organs. Further, the examination site discrimination unit 26 can also discriminate the examination site based on, for example, text information, a so-called schema image, or the like input from a user via the input device 31. Further, the examination site discrimination unit 26 can also sequentially recognize the types of sites of the subject captured in the ultrasound image U to discriminate the examination site based on the order of the recognized sites, for example, in a case where the subject is examined according to a predetermined examination protocol.

In step S4, the examination mode setting unit 27 sets the examination mode corresponding to the examination site discriminated in step S3. For example, the examination mode setting unit 27 can set the vein search mode as the examination mode in a case where the examination site is discriminated to be the upper limb, can set the thrombosis determination mode as the examination mode in a case where the examination site is discriminated to be the lower limb, and can set the elastic modulus measurement mode as the examination mode in a case where the examination site is discriminated to be the neck.

In step S5, the compression detection unit 25 detects the compression motion on the body surface of the subject by the ultrasound probe 1 based on, for example, the change in the diameter in the depth direction of the blood vessel B detected in step S2. In this case, the compression detection unit 25 can calculate, for example, the ratio R2/R1 between the diameter R1 in the depth direction of the blood vessel B in a state in which no compression is applied by the ultrasound probe 1 and the diameter R2 in the depth direction of the blood vessel B in a state in which compression is applied by the ultrasound probe 1 to detect the compression motion in a case where the ratio R2/R1 is equal to or less than a predetermined ratio threshold value.

In step S6, the compression motion determination unit 28 determines whether or not the compression motion detected in step S5 is proper for the examination mode set in step S4. In this case, the compression motion determination unit 28 has, for example, a predetermined allowable range of the compression motion for each examination mode, and determines whether or not the compression motion is proper by comparing the compression motion detected in step S5 with the allowable range corresponding to the examination mode set in step S4. As a specific example, the compression motion determination unit 28 can determine whether or not the compression motion is proper by using the ratio R2/R1 calculated in step S5 as the compression motion indicator representing the magnitude of the compression motion by the ultrasound probe 1 to compare the ratio R2/R1 with the predetermined proper range of values for each examination mode for the ratio R2/R1.

In step S7, the notification unit 29 notifies the user of the determination result in step S7, for example, by displaying the determination result on the monitor 23. The user can easily perform the compression motion suitable for the examination site by applying the compression to the body surface of the subject using the ultrasound probe 1 while confirming the determination result obtained through the notification in step S7.

In step S8, the main body controller 30 determines whether or not to end the examination. In this case, the main body controller 30 can determine to end the examination, for example, in a case where an instruction to end the examination is input from the user via the input device 31. Further, the main body controller 30 can determine to continue the examination, for example, in a case where no instruction to end the examination is input from the user via the input device 31.

In a case where it is determined in step S8 to continue the examination, the process returns to step S2, and processing of detecting the blood vessel B is performed on the continuously generated ultrasound images U. In a case where step S2 is completed, the examination site is newly discriminated in step S3. In subsequent step S4, in a case where the examination mode corresponding to the examination site discriminated in step S3 is different from the already set examination mode, the examination mode setting unit 27 sets a new examination mode corresponding to the examination site discriminated in step S3. Next, the compression motion by the ultrasound probe 1 is detected in step S5, it is determined in step S6 whether or not the compression motion is proper, and the user is notified of the determination result in step S7.

In this manner, the processing of steps S2 to S8 is repeated as long as it is determined in step S8 to continue the examination. In a case where the examination site is changed at this time, the examination mode corresponding to the new examination site is automatically set in step S4, and it is automatically determined in step S6 whether or not the compression motion by the ultrasound probe 1 is proper for this new examination mode. Therefore, the user can perform the compression motion suitable for the examination site while smoothly proceeding with the examination, for example, because there is no need to manually switch the examination modes even in a case where the examination site is changed in the middle of the examination.

In a case where it is determined in step S8 to end the examination, the operation of the ultrasound diagnostic apparatus according to the flowchart of Fig. 6 is completed.

From the above, with the ultrasound diagnostic apparatus of Embodiment 1, the examination site discrimination unit 26 automatically discriminates the examination site, the examination mode corresponding to the examination site discriminated by the examination mode setting unit 27 is automatically set, the compression detection unit 25 detects the compression motion on the body surface of the subject by the ultrasound probe 1, the compression motion determination unit 28 determines whether or not the compression motion is proper for the set examination mode, and the notification unit 29 notifies the user of the determination result. Therefore, the user can easily perform the compression motion suitable for the examination site by applying the compression to the body surface of the subject using the ultrasound probe 1 while confirming the determination result obtained through the notification from the notification unit 29 without the need to manually switch the examination modes even in a case where the examination site is changed in the middle of the examination. As a result, the user can smoothly and appropriately perform the examination.

Although it has been described that the transmission and reception circuit 12 is provided in the ultrasound probe 1, the transmission and reception circuit 12 may be provided in the apparatus main body 2.

In addition, although it has been described that the image generation unit 21 is provided in the apparatus main body 2, the image generation unit 21 may be provided in the ultrasound probe 1.

Further, the apparatus main body 2 may be a so-called stationary type, a portable type that is easy to carry, or a so-called handheld type that is composed of, for example, a smartphone or a tablet type computer. As described above, the type of the device that constitutes the apparatus main body 2 is not particularly limited.

Further, although it has been described that the notification unit 29 notifies the user by displaying the determination result of the compression motion determination unit 28 on the monitor 23, the method of notifying the user is not limited to this. For example, in a case where the ultrasound diagnostic apparatus comprises a speaker (not shown), the notification unit 29 can also notify the user of the determination result of the compression motion determination unit 28 by sound via the speaker.

### Embodiment 2

The ultrasound diagnostic apparatus can also perform the detection of the compression motion and the discrimination of the examination site based on an optical image obtained by capturing the subject and the ultrasound probe 1 during the examination.

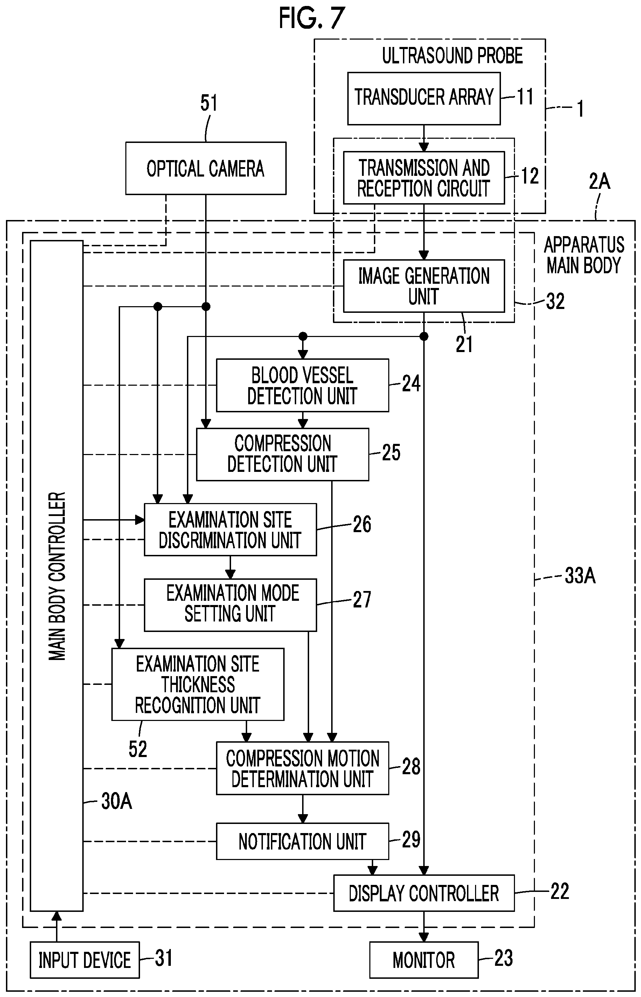

Fig. 7 shows a configuration of an ultrasound diagnostic apparatus of Embodiment 2. The ultrasound diagnostic apparatus of Embodiment 2 further comprises an optical camera 51 and comprises an apparatus main body 2A instead of the apparatus main body 2, with respect to the ultrasound diagnostic apparatus of Embodiment 1 shown in Fig. 1. The apparatus main body 2A further comprises an examination site thickness recognition unit 52 and comprises a main body controller 30A instead of the main body controller 30, with respect to the apparatus main body 2 in Embodiment 1.

In the ultrasound diagnostic apparatus of Embodiment 2, the compression detection unit 25, the examination site discrimination unit 26, the examination site thickness recognition unit 52, and the main body controller 30A are connected to the optical camera 51. The examination site thickness recognition unit 52 is connected to the compression motion determination unit 28 and the main body controller 30A. Further, the image generation unit 21, the display controller 22, the blood vessel detection unit 24, the compression detection unit 25, the examination site discrimination unit 26, the examination mode setting unit 27, the compression motion determination unit 28, the notification unit 29, the main body controller 30A, and the examination site thickness recognition unit 52 constitute a processor 33A for the apparatus main body 2A.

The optical camera 51 includes, for example, an image sensor, such as a so-called charge coupled device (CCD) image sensor or a so-called complementary metal-oxide-semiconductor (CMOS) image sensor, and images the body surface of the subject and the ultrasound probe 1 disposed on the body surface of the subject to acquire an optical image. The optical camera 51 sends out the acquired optical image to the compression detection unit 25 and the examination site thickness recognition unit 52.

The compression detection unit 25 can analyze the optical image acquired by the optical camera 51 to detect the compression motion based on a degree of depression of the body surface of the subject in the optical image or a movement of the arm of the user holding the ultrasound probe 1 and to calculate the compression motion indicator representing the magnitude of the compression motion. In this case, the compression detection unit 25 can detect the compression motion using, for example, a trained model that has learned in advance a relationship between the degree of depression of the body surface of the subject in the optical image and the compression motion, or a relationship between the movement of the arm of the user holding the ultrasound probe 1 and the compression motion.

The examination site discrimination unit 26 can discriminate the examination site based on the optical image acquired by the optical camera 51. In this case, the examination site discrimination unit 26 can discriminate the examination site by using a trained model that has learned in advance a combination of a large number of optical images showing the body surface of the subject and the ultrasound probe 1 and the corresponding examination site.

The examination mode setting unit 27 sets the examination mode corresponding to the examination site discriminated based on the optical image by the examination site discrimination unit 26 in this manner.

The compression motion determination unit 28 determines whether or not the compression motion is proper based on the compression motion detected by the compression detection unit 25 based on the optical image, and the examination mode set by the examination mode setting unit 27.

The examination site thickness recognition unit 52 recognizes the thickness of the examination site by analyzing the optical image acquired by the optical camera 51. In this case, the examination site thickness recognition unit 52 can recognize the thickness of the examination site by detecting, for example, the ultrasound probe 1 and the examination site shown in the optical image and by comparing the size of the ultrasound probe 1 and the size of the examination site with each other.

Here, in general, the thickness of the examination site is often proportional to the thickness of the blood vessel B at the examination site. In that respect, in a case of determining whether or not the compression motion is proper, the compression motion determination unit 28 can adjust the allowable range of the compression motion according to the thickness of the examination site recognized by the examination site thickness recognition unit 52. In this case, the examination site thickness recognition unit 52 can set a larger allowable range of the compression motion as the examination site is thicker, and can set a smaller allowable range of the compression motion as the examination site is thinner. As a result, the compression motion determination unit 28 can more accurately determine whether or not the compression motion is proper for the subject currently being examined.

From the above, with the ultrasound diagnostic apparatus of Embodiment 2, the compression motion determination unit 28 determines whether or not the compression motion is proper for the set examination mode, and the notification unit 29 notifies the user of the determination result, in the same manner as a case where the detection of the compression motion and the discrimination of the examination site are performed based on the ultrasound image U as in the ultrasound diagnostic apparatus of Embodiment 1 even in a case where the detection of the compression motion and the discrimination of the examination site are performed based on the optical image. Therefore, the user can smoothly and appropriately perform the examination. In addition, since the compression motion determination unit 28 can adjust the allowable range of the compression motion according to the thickness of the examination site recognized by the examination site thickness recognition unit 52 to improve the accuracy of the determination as to whether the compression motion is proper, the user can more accurately perform the examination.

### Embodiment 3

The ultrasound diagnostic apparatus of the embodiment of the present invention can also detect the compression motion by projecting a so-called moire pattern onto the body surface of the subject.

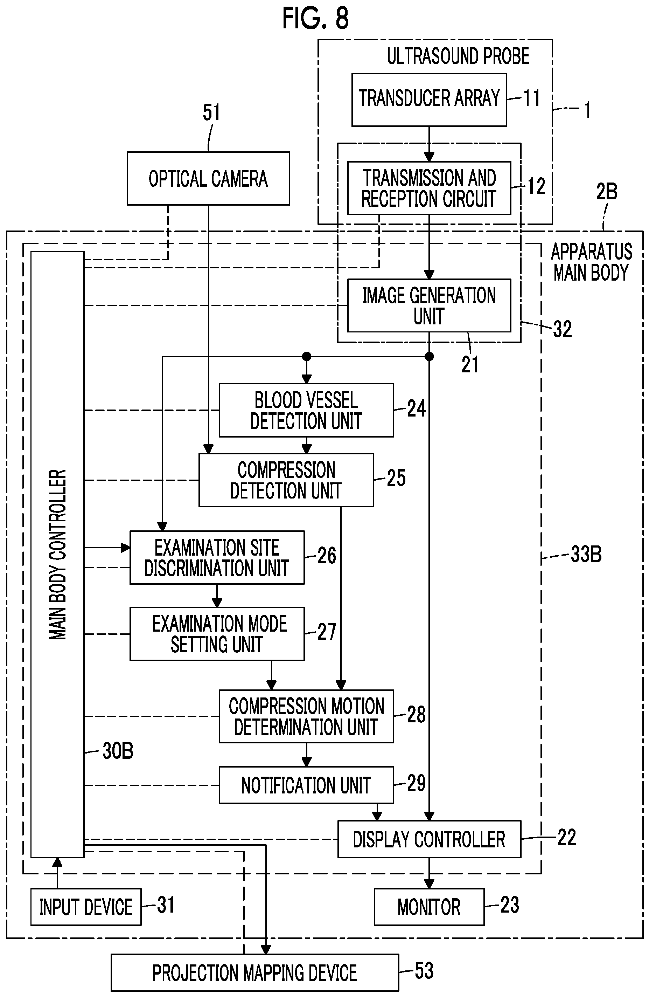

Fig. 8 shows a configuration of an ultrasound diagnostic apparatus of Embodiment 3. The ultrasound diagnostic apparatus of Embodiment 3 further comprises the optical camera 51 and a projection mapping device 53 and comprises an apparatus main body 2B instead of the apparatus main body 2, with respect to the ultrasound diagnostic apparatus of Embodiment 1 shown in Fig. 1. The apparatus main body 2B comprises a main body controller 30B instead of the main body controller 30 with respect to the apparatus main body 2 in Embodiment 1.

In the ultrasound diagnostic apparatus of Embodiment 3, the compression detection unit 25 and the main body controller 30B are connected to the optical camera 51. The projection mapping device 53 is connected to the main body controller 30B. Further, the image generation unit 21, the display controller 22, the blood vessel detection unit 24, the compression detection unit 25, the examination site discrimination unit 26, the examination mode setting unit 27, the compression motion determination unit 28, the notification unit 29, and the main body controller 30B constitute a processor 33B for the apparatus main body 2B.

The projection mapping device 53 is composed of a so-called projector and projects a moire pattern onto the body surface of the subject. In a case where the body surface of the subject is compressed by the ultrasound probe 1 in a state in which the moire pattern is projected onto the body surface of the subject in this manner, the shape of the moire pattern changes.

The optical camera 51 acquires the optical image showing the body surface of the subject on which the moire pattern is projected by the projection mapping device 53 and the ultrasound probe 1 disposed on the body surface of the subject.

The compression detection unit 25 can analyze a plurality of continuous frames of optical images acquired by the optical camera 51 to detect the compression motion based on the change in the moire pattern projected onto the body surface of the subject by the projection mapping device 53 and to calculate the compression motion indicator representing the magnitude of the compression motion. The compression detection unit 25 can detect the compression motion from the change in the moire pattern by using, for example, a trained model that has learned in advance a relationship between the change in the moire pattern projected onto the body surface of the subject and the compression motion.

The compression motion determination unit 28 determines whether or not the compression motion is proper based on the compression motion detected by the compression detection unit 25 based on the change in the moire pattern on the body surface of the subject in this manner, and the examination mode set by the examination mode setting unit 27.

The notification unit 29 notifies the user of the determination result by the compression motion determination unit 28.

From the above, with the ultrasound diagnostic apparatus of Embodiment 3, the compression motion determination unit 28 determines whether or not the compression motion is proper, and the notification unit 29 notifies the user of the determination result even in a case where the compression detection unit 25 detects the compression motion based on the change in the moire pattern projected onto the body surface of the subject. Therefore, the user can smoothly and appropriately perform the examination in the same manner as in the ultrasound diagnostic apparatus of Embodiment 1.

Although the ultrasound diagnostic apparatus of Embodiment 3 has a configuration in which the optical camera 51 and the projection mapping device 53 are added to the ultrasound diagnostic apparatus of Embodiment 1, a configuration can also be employed in which the projection mapping device 53 is added to the ultrasound diagnostic apparatus of Embodiment 2.

### Embodiment 4

The ultrasound diagnostic apparatus of the embodiment of the present invention can also detect the compression motion based on a change in an output value of a sensor attached to the ultrasound probe 1.

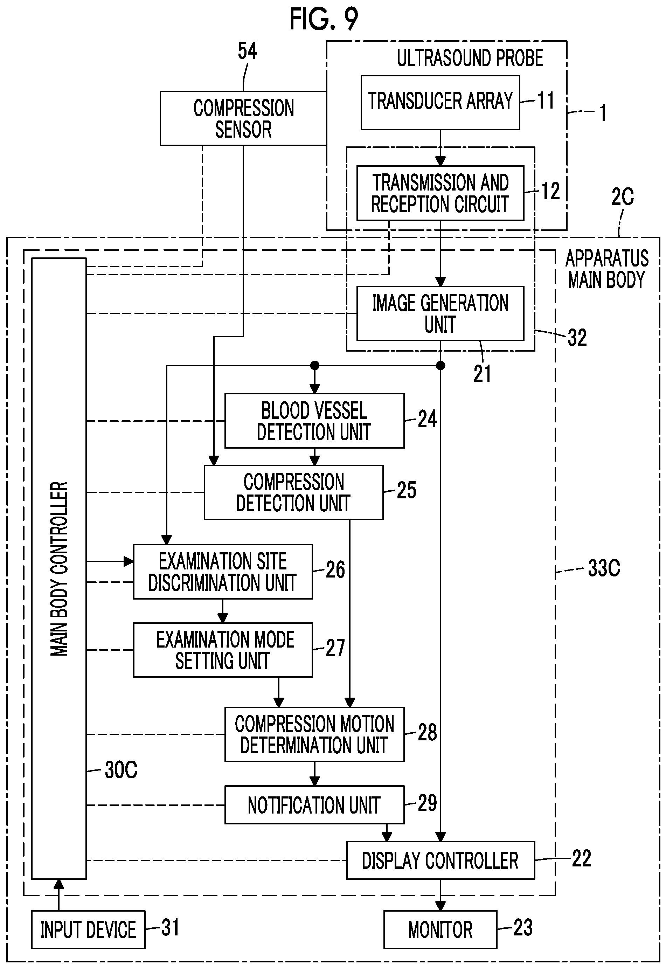

Fig. 9 shows a configuration of an ultrasound diagnostic apparatus of Embodiment 4. The ultrasound diagnostic apparatus of Embodiment 4 further comprises a compression sensor 54 attached to the ultrasound probe 1 and comprises an apparatus main body 2C instead of the apparatus main body 2, with respect to the ultrasound diagnostic apparatus of Embodiment 1 shown in Fig. 1. The apparatus main body 2C comprises a main body controller 30C instead of the main body controller 30 with respect to the apparatus main body 2 in Embodiment 1.

In the ultrasound diagnostic apparatus of Embodiment 4, the compression sensor 54 is connected to the compression detection unit 25 and the main body controller 30. Further, the image generation unit 21, the display controller 22, the blood vessel detection unit 24, the compression detection unit 25, the examination site discrimination unit 26, the examination mode setting unit 27, the compression motion determination unit 28, the notification unit 29, and the main body controller 30C constitute a processor 33C for the apparatus main body 2C.

The compression sensor 54 is composed of a pressure sensor that detects a pressure applied to a tip part of the ultrasound probe 1 or a so-called acceleration sensor, and changes the output value according to the compression motion on the body surface of the subject by the ultrasound probe 1.

The compression detection unit 25 detects the compression motion based on the change in the output value of the compression sensor 54 and calculates the compression motion indicator representing the magnitude of the compression motion.

The compression motion determination unit 28 determines whether or not the compression motion is proper based on the compression motion detected by the compression detection unit 25 based on the change in the output value of the compression sensor 54, and the examination mode set by the examination mode setting unit 27.

The notification unit 29 notifies the user of the determination result by the compression motion determination unit 28.

From the above, with the ultrasound diagnostic apparatus of Embodiment 4, the compression motion determination unit 28 determines whether or not the compression motion is proper, and the notification unit 29 notifies the user of the determination result even in a case where the compression detection unit 25 detects the compression motion based on the change in the output value of the compression sensor 54. Therefore, the user can smoothly and appropriately perform the examination in the same manner as in the ultrasound diagnostic apparatus of Embodiment 1.

Although the ultrasound diagnostic apparatus of Embodiment 4 has a configuration in which the compression sensor 54 is added to the ultrasound diagnostic apparatus of Embodiment 1, a configuration can also be employed in which the compression sensor 54 is added to the ultrasound diagnostic apparatus of Embodiment 2 and the ultrasound diagnostic apparatus of Embodiment 3.

### Embodiment 5

The ultrasound diagnostic apparatus of the embodiment of the present invention can also improve the accuracy of the examination by performing the processing corresponding to the specified subject and user.

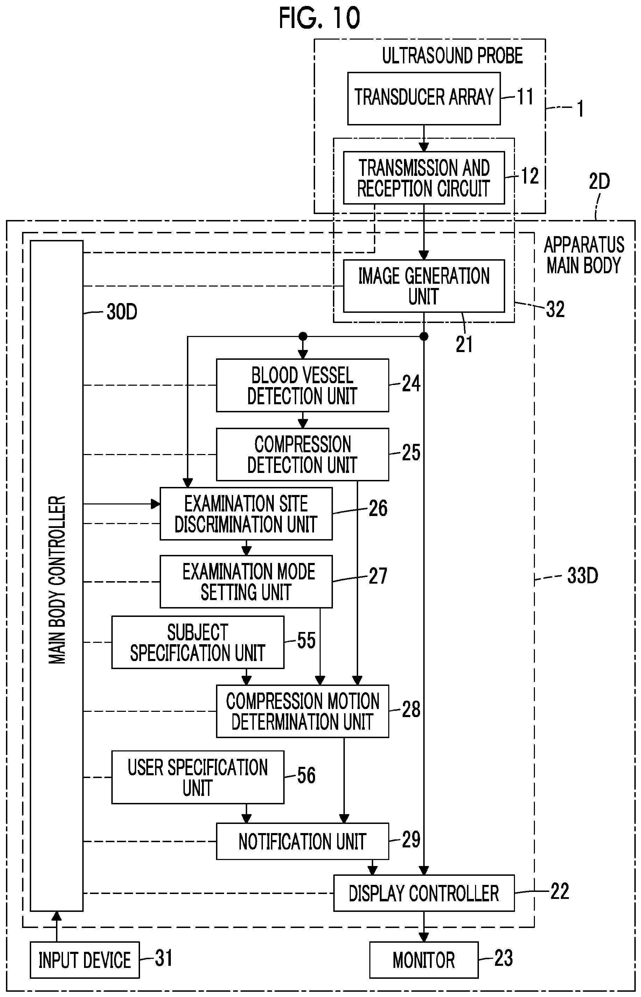

Fig. 10 shows a configuration of an ultrasound diagnostic apparatus of Embodiment 5. The ultrasound diagnostic apparatus of Embodiment 5 comprises an apparatus main body 2D instead of the apparatus main body 2 with respect to the ultrasound diagnostic apparatus of Embodiment 1 shown in Fig. 1. The apparatus main body 2D further comprises a subject specification unit 55 and a user specification unit 56 and comprises a main body controller 30D instead of the main body controller 30, with respect to the apparatus main body 2 in Embodiment 1.

In the apparatus main body 2D, the subject specification unit 55 is connected to the main body controller 30D. The subject specification unit 55 is connected to the compression motion determination unit 28. In addition, the user specification unit 56 is connected to the main body controller 30D. The user specification unit 56 is connected to the notification unit 29. Further, the image generation unit 21, the display controller 22, the blood vessel detection unit 24, the compression detection unit 25, the examination site discrimination unit 26, the examination mode setting unit 27, the compression motion determination unit 28, the notification unit 29, the main body controller 30D, the subject specification unit 55, and the user specification unit 56 constitute a processor 33D for the apparatus main body 2D.

The subject specification unit 55 stores identification information of a plurality of subjects and characteristics of each subject, such as the age and the sex, and specifies the subject based on the identification information of the subject input from the user via the input device 31.

The compression motion determination unit 28 adjusts the allowable range of the compression motion according to the age, the sex, and the like of the subject specified by the subject specification unit 55. Here, in general, as the muscle mass of the subject increases, a stronger force tends to be required in a case of compressing the body surface of the subject using the ultrasound probe 1. In addition, in general, younger individuals tend to have more muscle mass than older individuals, and males tend to have more muscle mass than females. Therefore, for example, the compression motion determination unit 28 can set a relatively large allowable range of the compression motion for the younger individuals and can set a relatively small allowable range of the compression motion for the older individuals. Further, for example, the compression motion determination unit 28 can set a relatively large allowable range of the compression motion for the males and can set a relatively small allowable range of the compression motion for the females. As a result, the compression motion determination unit 28 can improve the accuracy of determination as to whether or not the compression motion is proper.

The user specification unit 56 stores identification information of a plurality of users and characteristics of each user, such as the age, the sex, and the proficiency level, and specifies the user based on the identification information of the user input from the user via the input device 31.

The notification unit 29 notifies the user of a message corresponding to the user specified by the user specification unit 56. In general, older individuals tend to have weaker strength than younger individuals, and females tend to have weaker strength than males. Therefore, in a case where the user is an older individual or a female, the notification unit 29 can alert the user by displaying, for example, a message such as "Please apply a firm compression force" on the monitor 23. In addition, the notification unit 29 can display such a message on the monitor 23 in a case where the proficiency level of the user is lower than a certain level, and can also stop displaying the message in a case where the proficiency level of the user is equal to or higher than a certain level.

The user can accurately perform the compression motion by confirming the message displayed by the notification unit 29 in this manner.

From the above, with the ultrasound diagnostic apparatus of Embodiment 5, the compression motion determination unit 28 adjusts the allowable range of the compression motion according to the age, the sex, and the like of the subject specified by the subject specification unit 55. Therefore, the accuracy of the determination as to whether or not the compression motion is proper can be improved. In addition, since the notification unit 29 notifies the user of a message corresponding to the user specified by the user specification unit 56, the user can accurately perform the compression motion. In this manner, the accuracy of the examination can be improved.

Although the ultrasound diagnostic apparatus of Embodiment 5 has a configuration in which the subject specification unit 55 and the user specification unit 56 are added to the ultrasound diagnostic apparatus of Embodiment 1, a configuration can also be employed in which the subject specification unit 55 and the user specification unit 56 are added to the ultrasound diagnostic apparatuses of Embodiments 2 to 4.

### Embodiment 6

In many cases, users with a high proficiency level can determine whether or not the compression motion is proper on their own. In that respect, the ultrasound diagnostic apparatus of the embodiment of the present invention can perform or stop the determination as to whether or not the compression motion is proper according to the proficiency level of the user.

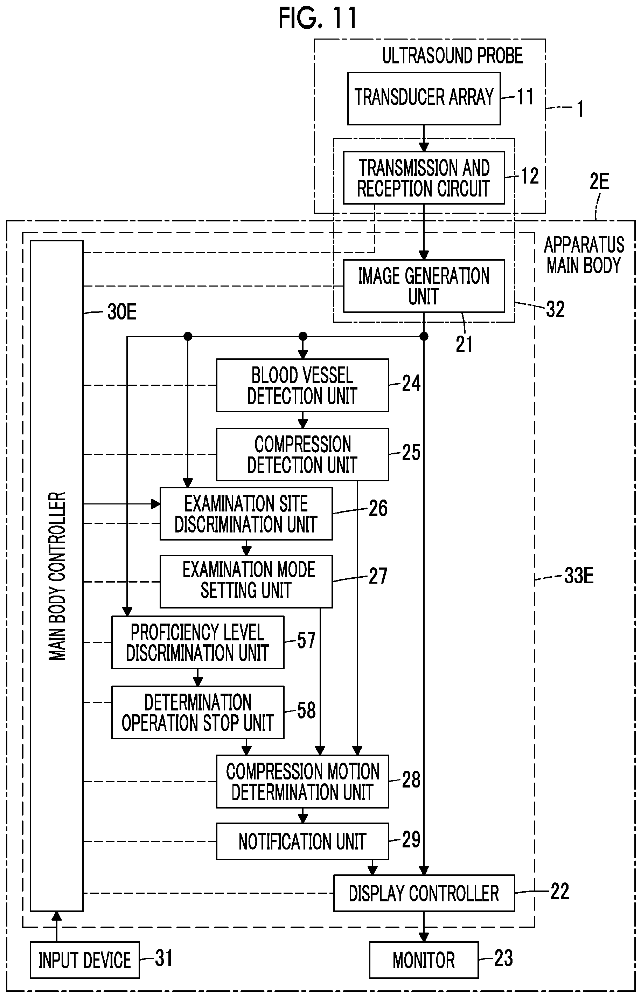

Fig. 11 shows a configuration of an ultrasound diagnostic apparatus of Embodiment 6. The ultrasound diagnostic apparatus of Embodiment 6 comprises an apparatus main body 2E instead of the apparatus main body 2 with respect to the ultrasound diagnostic apparatus of Embodiment 1 shown in Fig. 1. The apparatus main body 2E further comprises a proficiency level discrimination unit 57 and a determination operation stop unit 58 and comprises a main body controller 30E instead of the main body controller 30, with respect to the apparatus main body 2 in Embodiment 1.

In the apparatus main body 2E, the proficiency level discrimination unit 57 is connected to the image generation unit 21. The determination operation stop unit 58 and the main body controller 30E are connected to the proficiency level discrimination unit 57. The determination operation stop unit 58 is connected to the compression motion determination unit 28 and the main body controller 30E. Further, the image generation unit 21, the display controller 22, the blood vessel detection unit 24, the compression detection unit 25, the examination site discrimination unit 26, the examination mode setting unit 27, the compression motion determination unit 28, the notification unit 29, the main body controller 30E, the proficiency level discrimination unit 57, and the determination operation stop unit 58 constitute a processor 33E for the apparatus main body 2E.

The proficiency level discrimination unit 57 discriminates the proficiency level of the user by analyzing a plurality of continuous frames of ultrasound images U generated by the image generation unit 21. In a case where a user with a low proficiency level moves the ultrasound probe 1 to capture the ultrasound image U, for example, there may be occurrences such as obtaining an unclear image with an unstable cross section of the blood vessel B, and requiring a certain amount of time or longer for depicting the blood vessel B. Therefore, the proficiency level discrimination unit 57 can discriminate the proficiency level of the user based on, for example, the number of images clearly showing the blood vessel B among a certain number of continuous frames of ultrasound images U generated by the image generation unit 21, the time taken from the start of the examination until obtaining the ultrasound image U clearly showing the blood vessel B, or the like.

The determination operation stop unit 58 stops the operation of the determination by the compression motion determination unit 28 based on the proficiency level of the user discriminated by the proficiency level discrimination unit 57. In this case, the determination operation stop unit 58 can stop the operation of the determination by the compression motion determination unit 28, for example, in a case where the user is discriminated to have a certain proficiency level or higher by the proficiency level discrimination unit 57.

Here, in many cases, users with a high proficiency level can determine whether or not the compression motion is proper on their own. In this case, by stopping the operation of the determination by the compression motion determination unit 28, it is possible to save the calculation load and the power required for the processing of the compression motion determination unit 28.

From the above, with the ultrasound diagnostic apparatus of Embodiment 6, the proficiency level discrimination unit 57 automatically discriminates the proficiency level of the user, the determination operation stop unit 58 stops the operation of the determination by the compression motion determination unit 28 according to the discriminated proficiency level of the user. Therefore, it is possible to save the calculation load and the power required for the processing of the compression motion determination unit 28 particularly in a case where a user with a high proficiency level performs the examination.

Although it has been described that the proficiency level discrimination unit 57 discriminates the proficiency level of the user based on a plurality of continuous frames of ultrasound images U, the method of discriminating the proficiency level of the user is not limited to this. For example, in a case where the ultrasound diagnostic apparatus comprises the optical camera 51, the proficiency level discrimination unit 57 can discriminate the proficiency level of the user by analyzing a plurality of continuous frames of optical images acquired by the optical camera 51 and recognizing the user's technique. In this case, the proficiency level discrimination unit 57 can discriminate the proficiency level of the user from the plurality of continuous frames of optical images by using, for example, a trained model that has learned in advance a relationship between the user's technique represented by the plurality of continuous frames of optical images and the proficiency level of the user.

Although the ultrasound diagnostic apparatus of Embodiment 6 has a configuration in which the proficiency level discrimination unit 57 and the determination operation stop unit 58 are added to the ultrasound diagnostic apparatus of Embodiment 1, a configuration can also be employed in which the proficiency level discrimination unit 57 and the determination operation stop unit 58 are added to the ultrasound diagnostic apparatuses of Embodiments 2 to 5.

### Embodiment 7

In general, as the muscle mass of the subject increases, a stronger force tends to be required in a case of compressing the body surface of the subject using the ultrasound probe 1. In that respect, the ultrasound diagnostic apparatus of the embodiment of the present invention can also determine whether or not the compression motion is proper in consideration of the muscle mass of the subject.

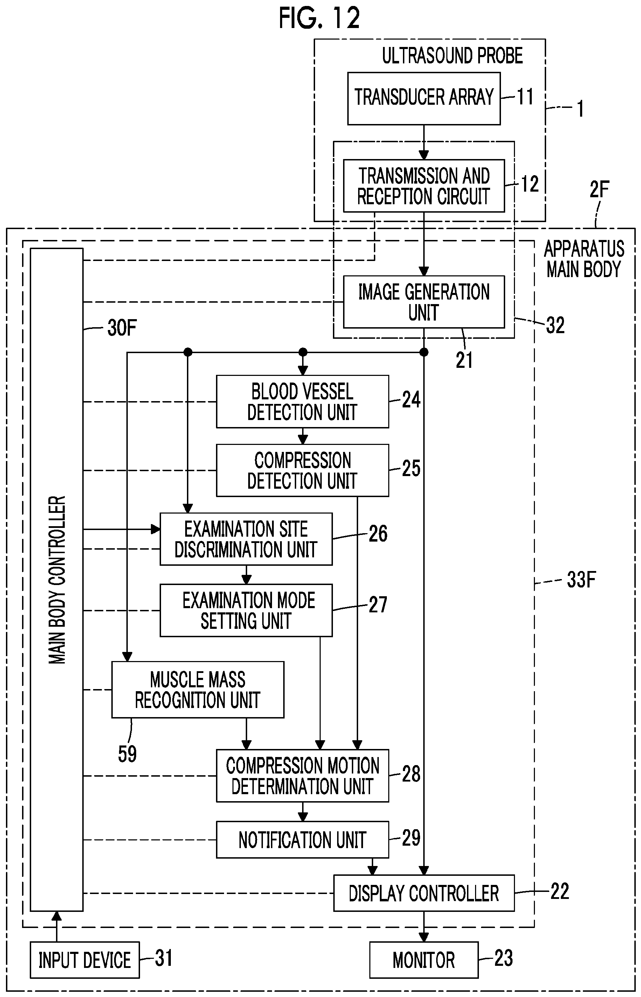

Fig. 12 shows a configuration of an ultrasound diagnostic apparatus of Embodiment 7. The ultrasound diagnostic apparatus of Embodiment 7 comprises an apparatus main body 2F instead of the apparatus main body 2 with respect to the ultrasound diagnostic apparatus of Embodiment 1 shown in Fig. 1. The apparatus main body 2F further comprises a muscle mass recognition unit 59 and comprises a main body controller 30F instead of the main body controller 30, with respect to the apparatus main body 2 in Embodiment 1.

In the apparatus main body 2F, the muscle mass recognition unit 59 is connected to the image generation unit 21. The muscle mass recognition unit 59 is connected to the compression motion determination unit 28 and the main body controller 30F. Further, the image generation unit 21, the display controller 22, the blood vessel detection unit 24, the compression detection unit 25, the examination site discrimination unit 26, the examination mode setting unit 27, the compression motion determination unit 28, the notification unit 29, the main body controller 30F, and the muscle mass recognition unit 59 constitute a processor 33F for the apparatus main body 2F.

The muscle mass recognition unit 59 recognizes the muscle mass of the subject by analyzing the ultrasound image U generated by the image generation unit 21. The muscle mass recognition unit 59 can recognize the muscle mass of the subject, for example, by detecting the muscle layer shown in the ultrasound image U using a template matching method or the like and measuring the thickness thereof. In addition, the muscle mass recognition unit 59 can also recognize the muscle mass of the subject from the ultrasound image U by using a trained model that has learned in advance a relationship between a large number of ultrasound images U showing the muscle layer of the subject and the muscle mass of the subject.

The compression motion determination unit 28 adjusts the allowable range of the compression motion according to the muscle mass of the subject recognized by the muscle mass recognition unit 59. In general, as the muscle mass of the subject increases, a stronger force is required in a case of compressing the body surface of the subject using the ultrasound probe 1. Therefore, for example, the compression motion determination unit 28 can set a larger allowable range of the compression motion as the muscle mass of the subject increases, and can set a smaller allowable range of the compression motion as the muscle mass of the subject decreases. As a result, the compression motion determination unit 28 can more accurately determine whether or not the compression motion is proper in consideration of the actual muscle mass of the subject.

From the above, with the ultrasound diagnostic apparatus of Embodiment 7, the muscle mass recognition unit 59 recognizes the muscle mass of the subject from the ultrasound image U, and the compression motion determination unit 28 adjusts the allowable range of the compression motion according to the muscle mass recognized by the muscle mass recognition unit 59. Therefore, the accuracy of the determination as to whether or not the compression motion is proper can be improved, and the user can smoothly and appropriately perform the examination.

Although the ultrasound diagnostic apparatus of Embodiment 7 has a configuration in which the muscle mass recognition unit 59 is added to the ultrasound diagnostic apparatus of Embodiment 1, a configuration can also be employed in which the muscle mass recognition unit 59 is added to the ultrasound diagnostic apparatuses of Embodiments 2 to 6.

### Embodiment 8

The ultrasound diagnostic apparatus of the embodiment of the present invention can also guide the user on a recommended compression motion based on the determination result of the compression motion determination unit 28 in order to enable the user to more smoothly perform the examination.

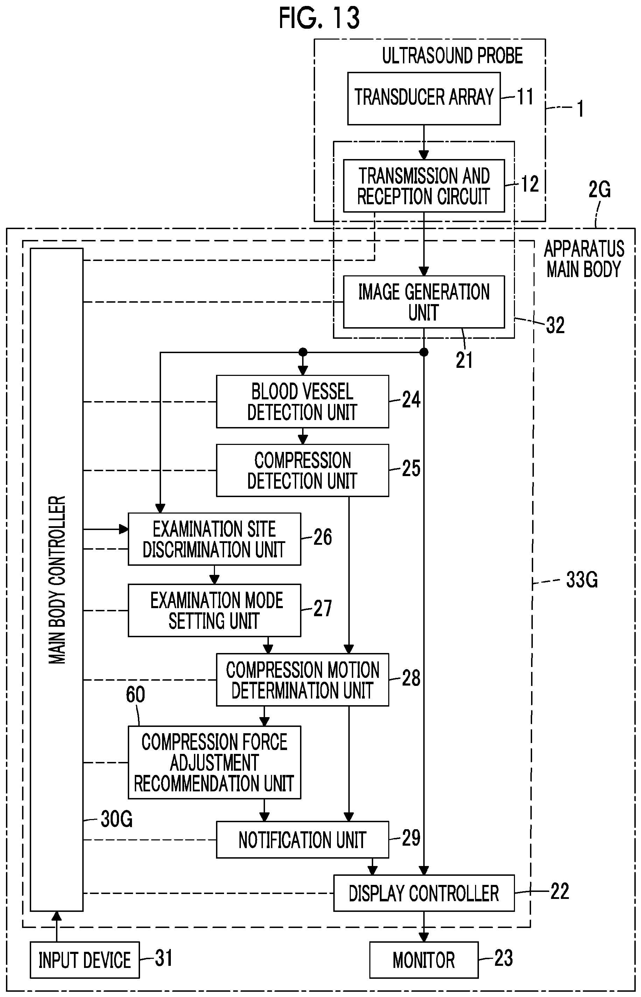

Fig. 13 shows a configuration of an ultrasound diagnostic apparatus of Embodiment 8. The ultrasound diagnostic apparatus of Embodiment 8 comprises an apparatus main body 2G instead of the apparatus main body 2 with respect to the ultrasound diagnostic apparatus of Embodiment 1 shown in Fig. 1. The apparatus main body 2G further comprises a compression force adjustment recommendation unit 60 and comprises a main body controller 30G instead of the main body controller 30, with respect to the apparatus main body 2 in Embodiment 1.

In the apparatus main body 2G, the compression force adjustment recommendation unit 60 is connected to the compression motion determination unit 28. The compression force adjustment recommendation unit 60 is connected to the notification unit 29 and the main body controller 30G. Further, the image generation unit 21, the display controller 22, the blood vessel detection unit 24, the compression detection unit 25, the examination site discrimination unit 26, the examination mode setting unit 27, the compression motion determination unit 28, the notification unit 29, the main body controller 30G, and the compression force adjustment recommendation unit 60 constitute a processor 33G for the apparatus main body 2G.

In a case where the compression motion is determined to be improper by the compression motion determination unit 28, the compression force adjustment recommendation unit 60 recommends adjusting the compression force of the ultrasound probe 1 on the body surface of the subject such that the compression motion falls within the allowable range. For example, in a case where the compression force by the ultrasound probe 1 exceeds the allowable range for the examination site and is strong, the compression force adjustment recommendation unit 60 can recommend reducing the compression force of the ultrasound probe 1. In addition, for example, in a case where the compression force by the ultrasound probe 1 falls below the allowable range for the examination site, the compression force adjustment recommendation unit 60 can recommend increasing the compression force of the ultrasound probe 1.

The notification unit 29 notifies the user of the adjustment of the compression force recommended by the compression force adjustment recommendation unit 60. The user can perform a proper compression motion for the examination site of the subject by confirming the adjustment of the compression force obtained through the notification from the notification unit 29 in this manner.

From the above, with the ultrasound diagnostic apparatus of Embodiment 8, in a case where the compression motion is determined to be improper by the compression motion determination unit 28, the compression force adjustment recommendation unit 60 recommends adjusting the compression force of the ultrasound probe 1 on the body surface of the subject such that the compression motion falls within the allowable range, and the notification unit 29 notifies the user of the adjustment of the compression force recommended by the compression force adjustment recommendation unit 60. Therefore, the user can improve the accuracy of the examination by performing a proper compression motion.

Although the ultrasound diagnostic apparatus of Embodiment 8 has a configuration in which the compression force adjustment recommendation unit 60 is added to the ultrasound diagnostic apparatus of Embodiment 1, a configuration can also be employed in which the compression force adjustment recommendation unit 60 is added to the ultrasound diagnostic apparatuses of Embodiments 2 to 7.

### Explanation of References

1: ultrasound probe
2, 2A to 2G: apparatus main body
11: transducer array
12: transmission and reception circuit
21: image generation unit
22: display controller
23: monitor
24: blood vessel detection unit
25: compression detection unit
26: examination site discrimination unit
27: examination mode setting unit
28: compression motion determination unit
29: notification unit
30, 30A to 30G: main body controller
31: input device
32: image acquisition unit
33, 33A to 33G: processor
41: pulsar
42: amplification section
43: AD conversion section
44: beam former
45: signal processing section
46: DSC
47: image processing section
51: optical camera
52: examination site thickness recognition unit
53: projection mapping device
54: compression sensor
55: subject specification unit
56: user specification unit
57: proficiency level discrimination unit
58: determination operation stop unit
59: muscle mass recognition unit
60: compression force adjustment recommendation unit
B: blood vessel
R1, R2: diameter
U: ultrasound image

## Claims

1. An ultrasound diagnostic apparatus that examines a blood vessel of a subject, the ultrasound diagnostic apparatus comprising:
an ultrasound probe (1);
an image acquisition unit (32) configured to continuously acquire an ultrasound image of the blood vessel using the ultrasound probe (1);
a blood vessel detection unit (24) configured to detect the blood vessel from the ultrasound image;
an examination site discrimination unit (26) configured to discriminate an examination site;
an examination mode setting unit (27) configured to set an examination mode corresponding to the examination site discriminated by the examination site discrimination unit (26);
a compression detection unit (25) configured to detect a compression motion on a body surface of the subject by the ultrasound probe (1);
a compression motion determination unit (28) configured to determine whether or not the compression motion detected by the compression detection unit (25) is proper for the examination mode set by the examination mode setting unit (27); and
a notification unit (29) configured to notify a user of a determination result by the compression motion determination unit (28).

2. The ultrasound diagnostic apparatus according to claim 1,
wherein the examination site discrimination unit (26) is configured to discriminate the examination site based on the ultrasound image.

3. The ultrasound diagnostic apparatus according to claim 1 or 2, further comprising:
an optical camera (51) configured to image the subject and the ultrasound probe (1) to acquire an optical image,
wherein the examination site discrimination unit (26) is configured to discriminate the examination site based on the optical image.

4. The ultrasound diagnostic apparatus according to any one of claims 1 to 3,
wherein the examination mode setting unit (27) is configured to set a vein search mode in a case where the examination site is an upper limb.

5. The ultrasound diagnostic apparatus according to any one of claims 1 to 4,
wherein the examination mode setting unit (27) is configured to set a thrombosis determination mode in a case where the examination site is a lower limb.

6. The ultrasound diagnostic apparatus according to any one of claims 1 to 5,
wherein the examination mode setting unit (27) is configured to set an elastic modulus measurement mode in a case where the examination site is a neck.

7. The ultrasound diagnostic apparatus according to any one of claims 1 to 6,
wherein the compression detection unit (25) is configured to detect the compression motion based on a change in a diameter in a depth direction of the blood vessel in the ultrasound image.

8. The ultrasound diagnostic apparatus according to claim 1 or 2, further comprising:
an optical camera (51) configured to image the subject and the ultrasound probe (1) to acquire an optical image,
wherein the compression detection unit (25) is configured to detect the compression motion based on a degree of depression of the body surface of the subject in the optical image or a movement of an arm of the user.

9. The ultrasound diagnostic apparatus according to any one of claims 1 to 6, further comprising:
a projection mapping device (53) configured to project a moire pattern onto the body surface of the subject,
wherein the compression detection unit (25) is configured to detect the compression motion based on a change in the moire pattern projected onto the body surface of the subject.

10. The ultrasound diagnostic apparatus according to any one of claims 1 to 6, further comprising:
a pressure sensor or an acceleration sensor attached to the ultrasound probe (1),
wherein the compression detection unit (25) is configured to detect the compression motion based on a change in an output value of the pressure sensor or the acceleration sensor.

11. The ultrasound diagnostic apparatus according to any one of claims 1 to 10,
wherein the compression motion determination unit (28) has a predetermined allowable range of the compression motion for each examination mode and is configured to determine whether or not the compression motion is proper by comparing the compression motion detected by the compression detection unit (25) with the allowable range corresponding to the examination mode set by the examination mode setting unit (27).

12. The ultrasound diagnostic apparatus according to claim 11, further comprising:
a subject specification unit (55) configured to specify the subject in response to an input of identification information of the subject,
wherein the compression motion determination unit (28) is configured to adjust the allowable range according to age and sex of the subject specified by the subject specification unit (55).

13. The ultrasound diagnostic apparatus according to claim 11, further comprising:
a muscle mass recognition unit (59) configured to recognize a muscle mass of the subject from the ultrasound image,
wherein the compression motion determination unit (28) is configured to adjust the allowable range according to the muscle mass recognized by the muscle mass recognition unit (59).

14. The ultrasound diagnostic apparatus according to claim 11, further comprising:
an optical camera (51) configured to image the subject to acquire an optical image; and
an examination site thickness recognition unit (52) configured to recognize a thickness of the examination site from the optical image acquired by the optical camera (51),
wherein the compression motion determination unit (28) is configured to adjust the allowable range according to the thickness of the examination site recognized by the examination site thickness recognition unit (52).

15. The ultrasound diagnostic apparatus according to any one of claims 11 to 14, further comprising:
a compression force adjustment recommendation unit (60) configured to, in a case where the compression motion is determined to be improper by the compression motion determination unit (28), recommend adjusting a compression force of the ultrasound probe (1) on the body surface of the subject such that the compression motion falls within the allowable range,
wherein the notification unit (29) is configured to notify the user of the adjustment of the compression force recommended by the compression force adjustment recommendation unit (60).

16. The ultrasound diagnostic apparatus according to any one of claims 1 to 15, further comprising:
a proficiency level discrimination unit (57) configured to discriminate a proficiency level of the user; and
a determination operation stop unit (58) configured to stop an operation of determination by the compression motion determination unit (28) according to the proficiency level of the user discriminated by the proficiency level discrimination unit (57).

17. The ultrasound diagnostic apparatus according to any one of claims 1 to 16, further comprising:
a user specification unit (56) configured to specify the user in response to an input of identification information of the user,
wherein the notification unit (29) is configured to notify the user of a message corresponding to the user specified by the user specification unit (56).

18. A control method of an ultrasound diagnostic apparatus that examines a blood vessel of a subject, the control method comprising:
continuously acquiring an ultrasound image of the blood vessel using an ultrasound probe (1);
detecting the blood vessel from the ultrasound image;
discriminating an examination site;
setting an examination mode corresponding to the discriminated examination site;
detecting a compression motion on a body surface of the subject by the ultrasound probe (1);
determining whether or not the compression motion detected for the set examination mode is proper; and
notifying a user of a determination result.

## Patentansprüche

1. Ultraschalldiagnosevorrichtung, die ein Blutgefäß einer Untersuchungsperson untersucht, wobei die Ultraschalldiagnosevorrichtung umfasst:
eine Ultraschallsonde (1);
eine Bilderfassungseinheit (32), die so konfiguriert ist, dass sie ein Ultraschallbild des Blutgefäßes unter Verwendung der Ultraschallsonde (1) kontinuierlich erfasst;
eine Blutgefäß-Detektionseinheit (24), die so konfiguriert ist, dass sie das Blutgefäß aus dem Ultraschallbild detektiert;
eine Untersuchungsstellen-Diskriminierungseinheit (26), die so konfiguriert ist, dass sie eine Untersuchungsstelle diskriminiert;
eine Untersuchungsmodus-Einstelleinheit (27), die so konfiguriert ist, dass sie einen Untersuchungsmodus, der der Untersuchungsstelle entspricht, die von der Untersuchungsstellen-Diskriminierungseinheit (26) diskriminiert wurde, einstellt;
eine Kompressionsdetektionseinheit (25), die so konfiguriert ist, dass sie eine Kompressionsbewegung auf einer Körperoberfläche der Untersuchungsperson durch die Ultraschallsonde (1) detektiert;
eine Kompressionsbewegungs-Bestimmungseinheit (28), die so konfiguriert ist, dass sie bestimmt, ob die von der Kompressionsdetektionseinheit (25) detektierte Kompressionsbewegung für den von der Untersuchungsmodus-Einstelleinheit (27) eingestellten Untersuchungsmodus ordnungsgemäß ist oder nicht; und
eine Benachrichtigungseinheit (29), die so konfiguriert ist, dass sie einen Benutzer über ein Bestimmungsergebnis durch die Kompressionsbewegungs-Bestimmungseinheit (28) benachrichtigt.

2. Ultraschalldiagnosevorrichtung nach Anspruch 1,
wobei die Untersuchungsstellen-Diskriminierungseinheit (26) so konfiguriert ist, dass sie die Untersuchungsstelle auf der Grundlage des Ultraschallbildes diskriminiert.

3. Ultraschalldiagnosevorrichtung nach Anspruch 1 oder 2, ferner umfassend:
eine optische Kamera (51), die so konfiguriert ist, dass sie die Untersuchungsperson abbildet, und die Ultraschallsonde (1), um ein optisches Bild zu erfassen,
wobei die Untersuchungsstellen-Diskriminierungseinheit (26) so konfiguriert ist, dass sie die Untersuchungsstelle auf der Grundlage des optischen Bildes diskriminiert.

4. Ultraschalldiagnosevorrichtung nach einem der Ansprüche 1 bis 3,
wobei die Untersuchungsmodus-Einstelleinheit (27) so konfiguriert ist, dass sie einen Venensuchmodus in einem Fall einstellt, in dem die Untersuchungsstelle ein oberes Gliedmaß ist.

5. Ultraschalldiagnosevorrichtung nach einem der Ansprüche 1 bis 4,
wobei die Untersuchungsmodus-Einstelleinheit (27) so konfiguriert ist, dass sie einen Thrombose-Bestimmungsmodus in einem Fall einstellt, in dem die Untersuchungsstelle ein unteres Gliedmaß ist.

6. Ultraschalldiagnosevorrichtung nach einem der Ansprüche 1 bis 5,
wobei die Untersuchungsmodus-Einstelleinheit (27) so konfiguriert ist, dass sie einen Elastizitätsmodul-Messmodus in einem Fall einstellt, in dem die Untersuchungsstelle ein Hals ist.

7. Ultraschalldiagnosevorrichtung nach einem der Ansprüche 1 bis 6,
wobei die Kompressionsdetektionseinheit (25) so konfiguriert ist, dass sie die Kompressionsbewegung auf der Grundlage einer Änderung eines Durchmessers in einer Tiefenrichtung des Blutgefäßes in dem Ultraschallbild detektiert.

8. Ultraschalldiagnosevorrichtung nach Anspruch 1 oder 2, ferner umfassend:
eine optische Kamera (51), die so konfiguriert ist, dass sie die Untersuchungsperson abbildet, und die Ultraschallsonde (1), um ein optisches Bild zu erfassen,
wobei die Kompressionsdetektionseinheit (25) so konfiguriert ist, dass sie die Kompressionsbewegung auf der Grundlage eines Grades an Eindrücktiefe der Körperoberfläche der Untersuchungsperson in dem optischen Bild oder einer Bewegung eines Arms des Benutzers detektiert.

9. Ultraschalldiagnosevorrichtung nach einem der Ansprüche 1 bis 6, ferner umfassend:
eine Projektionsmapping-Vorrichtung (53), die so konfiguriert ist, dass sie ein Moiré-Muster auf die Körperoberfläche der Untersuchungsperson projiziert,
wobei die Kompressionsdetektionseinheit (25) so konfiguriert ist, dass sie die Kompressionsbewegung auf der Grundlage einer Änderung des Moiré-Musters, das auf die Körperoberfläche der Untersuchungsperson projiziert wird, detektiert.

10. Ultraschalldiagnosevorrichtung nach einem der Ansprüche 1 bis 6, ferner umfassend:
einen Drucksensor oder einen Beschleunigungssensor, der an der Ultraschallsonde (1) angebracht ist,
wobei die Kompressionsdetektionseinheit (25) so konfiguriert ist, dass sie die Kompressionsbewegung auf der Grundlage einer Änderung eines Ausgabewerts des Drucksensors oder des Beschleunigungssensors detektiert.

11. Ultraschalldiagnosevorrichtung nach einem der Ansprüche 1 bis 10,
wobei die Kompressionsbewegungs-Bestimmungseinheit (28) einen vorbestimmten zulässigen Bereich der Kompressionsbewegung für jeden Untersuchungsmodus aufweist und so konfiguriert ist, dass sie durch Vergleichen der von der Kompressionsdetektionseinheit (25) detektierten Kompressionsbewegung mit dem zulässigen Bereich, der dem von der Untersuchungsmodus-Einstelleinheit (27) eingestellten Untersuchungsmodus entspricht, bestimmt, ob die Kompressionsbewegung ordnungsgemäß ist oder nicht.

12. Ultraschalldiagnosevorrichtung nach Anspruch 11, ferner umfassend:
eine Untersuchungsperson-Spezifikationseinheit (55), die so konfiguriert ist, dass sie die Untersuchungsperson als Reaktion auf eine Eingabe von Identifikationsinformationen der Untersuchungsperson spezifiziert,
wobei die Kompressionsbewegungs-Bestimmungseinheit (28) so konfiguriert ist, dass sie den zulässigen Bereich gemäß dem Alter und Geschlecht der Untersuchungsperson, die von der Untersuchungsperson-Spezifikationseinheit (55) spezifiziert wurden, anpasst.

13. Ultraschalldiagnosevorrichtung nach Anspruch 11, ferner umfassend:
eine Muskelmassen-Erkennungseinheit (59), die so konfiguriert ist, dass sie eine Muskelmasse der Untersuchungsperson aus dem Ultraschallbild erkennt,
wobei die Kompressionsbewegungs-Bestimmungseinheit (28) so konfiguriert ist, dass sie den zulässigen Bereich gemäß der von der Muskelmassen-Erkennungseinheit (59) erkannten Muskelmasse anpasst.

14. Ultraschalldiagnosevorrichtung nach Anspruch 11, ferner umfassend:
eine optische Kamera (51), die so konfiguriert ist, dass sie die Untersuchungsperson abbildet, um ein optisches Bild zu erfassen; und
eine Untersuchungstellendicke-Erkennungseinheit (52), die so konfiguriert ist, dass sie eine Dicke der Untersuchungstelle aus dem optischen Bild, das von der optischen Kamera (51) erfasst wurde, erkennt,
wobei die Kompressionsbewegungs-Bestimmungseinheit (28) so konfiguriert ist, dass sie den zulässigen Bereich gemäß der Dicke der Untersuchungsstelle, die von der Untersuchungsstellendicke-Erkennungseinheit (52) erkannt wurde, anpasst.

15. Ultraschalldiagnosevorrichtung nach einem der Ansprüche 11 bis 14, ferner umfassend:
eine Kompressionskraftanpassungs-Empfehlungseinheit (60), die so konfiguriert ist, dass sie in einem Fall, in dem durch die Kompressionsbewegungs-Bestimmungseinheit (28) bestimmt wird, dass die Kompressionsbewegung nicht ordnungsgemäß ist, Anpassen einer Kompressionskraft der Ultraschallsonde (1) auf der Körperoberfläche der Untersuchungsperson so empfiehlt, dass die Kompressionsbewegung innerhalb des zulässigen Bereichs liegt,
wobei die Benachrichtigungseinheit (29) so konfiguriert ist, dass sie den Benutzer über die Anpassung der von der Kompressionskraftanpassungs-Empfehlungseinheit (60) empfohlenen Kompressionskraft benachrichtigt.

16. Ultraschalldiagnosevorrichtung nach einem der Ansprüche 1 bis 15, ferner umfassend:
eine Kompetenzniveau-Diskriminierungseinheit (57), die so konfiguriert ist, dass sie ein Kompetenzniveau des Benutzers diskriminiert; und
eine Bestimmungsvorgang-Stoppeinheit (58), die so konfiguriert ist, dass sie einen Bestimmungsvorgang durch die Kompressionsbewegungs-Bestimmungseinheit (28) gemäß dem von der Kompetenzniveau-Diskriminierungseinheit (57) diskriminierten Kompetenzniveau des Benutzers stoppt.

17. Ultraschalldiagnosevorrichtung nach einem der Ansprüche 1 bis 16, ferner umfassend:
eine Benutzerspezifikationseinheit (56), die so konfiguriert ist, dass sie den Benutzer als Reaktion auf eine Eingabe von Identifikationsinformationen des Benutzers spezifiziert,
wobei die Benachrichtigungseinheit (29) so konfiguriert ist, dass sie den Benutzer über eine Nachricht, die dem Benutzer entspricht, der von der Benutzerspezifikationseinheit (56) spezifiziert wurde, benachrichtigt.

18. Steuerverfahren einer Ultraschalldiagnosevorrichtung, die ein Blutgefäß einer Untersuchungsperson untersucht, wobei das Steuerverfahren umfasst:
kontinuierliches Erfassen eines Ultraschallbildes des Blutgefäßes unter Verwendung einer Ultraschallsonde (1);
Detektieren des Blutgefäßes aus dem Ultraschallbild;
Diskriminieren einer Untersuchungsstelle;
Einstellen eines Untersuchungsmodus, der der diskriminierten Untersuchungsstelle entspricht;
Detektieren einer Kompressionsbewegung auf einer Körperoberfläche der Untersuchungsperson durch die Ultraschallsonde (1);
Bestimmen, ob die für den eingestellten Untersuchungsmodus detektierte Kompressionsbewegung ordnungsgemäß ist oder nicht; und
Benachrichtigen eines Benutzers über ein Bestimmungsergebnis.

## Revendications

1. Appareil de diagnostic par ultrasons qui examine un vaisseau sanguin d'un sujet, l'appareil de diagnostic par ultrasons comprenant :
une sonde ultrasonore (1) ;
une unité d'acquisition d'images (32) configurée pour acquérir en continu une image ultrasonore du vaisseau sanguin en utilisant la sonde ultrasonore (1) ;
une unité de détection des vaisseaux sanguins (24) configurée pour détecter le vaisseau sanguin à partir de l'image ultrasonore ;
une unité de discrimination de site d'examen (26) configurée pour discriminer un site d'examen ;
une unité de réglage de mode d'examen (27) configurée pour régler un mode d'examen correspondant au site d'examen discriminé par l'unité de discrimination de site d'examen (26) ;
une unité de détection de compression (25) configurée pour détecter un mouvement de compression sur une surface corporelle du sujet par la sonde ultrasonore (1) ;
une unité de détermination de mouvement de compression (28) configurée pour déterminer si oui ou non le mouvement de compression détecté par l'unité de détection de compression (25) est approprié pour le mode d'examen réglé par l'unité de réglage de mode d'examen (27) ; et
une unité de notification (29) configurée pour notifier un utilisateur d'un résultat de détermination par l'unité de détermination de mouvement de compression (28).

2. Appareil de diagnostic par ultrasons selon la revendication 1,
dans lequel l'unité de discrimination de site d'examen (26) est configurée pour discriminer le site d'examen sur la base de l'image ultrasonore.

3. Appareil de diagnostic par ultrasons selon la revendication 1 ou la revendication 2, comprenant en outre :
une caméra optique (51) configurée pour imager le sujet et la sonde ultrasonore (1) pour acquérir une image optique,
dans lequel l'unité de discrimination de site d'examen (26) est configurée pour discriminer le site d'examen sur la base de l'image optique.

4. Appareil de diagnostic par ultrasons selon l'une quelconque des revendications 1 à 3, dans lequel l'unité de réglage de mode d'examen (27) est configurée pour régler un mode de recherche de veine dans un cas où le site d'examen est un membre supérieur.

5. Appareil de diagnostic par ultrasons selon l'une quelconque des revendications 1 à 4, dans lequel l'unité de réglage de mode d'examen (27) est configurée pour régler un mode de détermination de thrombose dans un cas où le site d'examen est un membre inférieur.

6. Appareil de diagnostic par ultrasons selon l'une quelconque des revendications 1 à 5, dans lequel l'unité de réglage de mode d'examen (27) est configurée pour régler un mode de mesure de module élastique dans un cas où le site d'examen est un cou.

7. Appareil de diagnostic par ultrasons selon l'une quelconque des revendications 1 à 6, dans lequel l'unité de détection de compression (25) est configurée pour détecter le mouvement de compression sur la base d'un changement de diamètre dans une direction de profondeur du vaisseau sanguin dans l'image ultrasonore.

8. Appareil de diagnostic par ultrasons selon la revendication 1 ou la revendication 2, comprenant en outre :
une caméra optique (51) configurée pour imager le sujet et la sonde ultrasonore (1) pour acquérir une image optique,
dans lequel l'unité de détection de compression (25) est configurée pour détecter le mouvement de compression sur la base d'un degré de dépression de la surface corporelle du sujet dans l'image optique ou d'un mouvement d'un bras de l'utilisateur.

9. Appareil de diagnostic par ultrasons selon l'une quelconque des revendications 1 à 6, comprenant en outre :
un dispositif de cartographie par projection (53) configuré pour projeter un motif de moiré sur la surface corporelle du sujet,
dans lequel l'unité de détection de compression (25) est configurée pour détecter le mouvement de compression sur la base d'un changement du motif de moiré projeté sur la surface corporelle du sujet.

10. Appareil de diagnostic par ultrasons selon l'une quelconque des revendications 1 à 6, comprenant en outre :
un capteur de pression ou un capteur d'accélération fixé à la sonde ultrasonore (1),
dans lequel l'unité de détection de compression (25) est configurée pour détecter le mouvement de compression sur la base d'un changement d'une valeur de sortie du capteur de pression ou du capteur d'accélération.

11. Appareil de diagnostic par ultrasons selon l'une quelconque des revendications 1 à 10, dans lequel l'unité de détermination de mouvement de compression (28) a une plage admissible prédéterminée du mouvement de compression pour chaque mode d'examen et est configurée pour déterminer si oui ou non le mouvement de compression est approprié en comparant le mouvement de compression détecté par l'unité de détection de compression (25) avec la plage admissible correspondant au mode d'examen réglé par l'unité de réglage de mode d'examen (27).

12. Appareil de diagnostic par ultrasons selon la revendication 11, comprenant en outre :
une unité de spécification de sujet (55) configurée pour spécifier le sujet en réponse à une entrée d'informations d'identification du sujet,
dans lequel l'unité de détermination de mouvement de compression (28) est configurée pour ajuster la plage acceptable en fonction de l'âge et du sexe du sujet spécifié par l'unité de spécification de sujet (55).

13. Appareil de diagnostic par ultrasons selon la revendication 11, comprenant en outre :
une unité de reconnaissance de masse musculaire (59) configurée pour reconnaître une masse musculaire du sujet à partir de l'image ultrasonore,
dans lequel l'unité de détermination de mouvement de compression (28) est configurée pour ajuster la plage acceptable en fonction de la masse musculaire reconnue par l'unité de reconnaissance de masse musculaire (59).

14. Appareil de diagnostic par ultrasons selon la revendication 11, comprenant en outre :
une caméra optique (51) configurée pour imager le sujet afin d'acquérir une image optique ; et
une unité de reconnaissance d'épaisseur de site d'examen (52) configurée pour reconnaître une épaisseur du site d'examen à partir de l'image optique acquise par la caméra optique (51),
dans lequel l'unité de détermination de mouvement de compression (28) est configurée pour ajuster la plage acceptable en fonction de l'épaisseur du site d'examen reconnue par l'unité de reconnaissance d'épaisseur de site d'examen (52).

15. Appareil de diagnostic par ultrasons selon l'une quelconque des revendications 11 à 14, comprenant en outre :
une unité de recommandation d'ajustement de force de compression (60) configurée pour, dans un cas où le mouvement de compression est déterminé comme étant inapproprié par l'unité de détermination de mouvement de compression (28), recommander d'ajuster une force de compression de la sonde ultrasonore (1) sur la surface corporelle du sujet de sorte que le mouvement de compression se situe dans la plage acceptable,
dans lequel l'unité de notification (29) est configurée pour notifier l'utilisateur de l'ajustement de la force de compression recommandée par l'unité de recommandation d'ajustement de force de compression (60).

16. Appareil de diagnostic par ultrasons selon l'une quelconque des revendications 1 à 15, comprenant en outre :
une unité de discrimination de niveau de compétence (57) configurée pour discriminer un niveau de compétence de l'utilisateur ; et
une unité d'arrêt d'opération de détermination (58) configurée pour arrêter une opération de détermination par l'unité de détermination de mouvement de compression (28) en fonction du niveau de compétence de l'utilisateur discriminé par l'unité de discrimination de niveau de compétence (57).

17. Appareil de diagnostic par ultrasons selon l'une quelconque des revendications 1 à 16, comprenant en outre :
une unité de spécification d'utilisateur (56) configurée pour spécifier l'utilisateur en réponse à une entrée d'informations d'identification de l'utilisateur,
dans lequel l'unité de notification (29) est configurée pour notifier l'utilisateur d'un message correspondant à l'utilisateur spécifié par l'unité de spécification d'utilisateur (56).

18. Procédé de contrôle d'un appareil de diagnostic par ultrasons qui examine un vaisseau sanguin d'un sujet, le procédé de contrôle comprenant :
acquérir en continu une image ultrasonore du vaisseau sanguin en utilisant une sonde ultrasonore (1) ;
détecter le vaisseau sanguin à partir de l'image ultrasonore ;
discriminer un site d'examen ;
régler un mode d'examen correspondant au site d'examen discriminé ;
détecter un mouvement de compression sur une surface corporelle du sujet par la sonde ultrasonore (1) ;
déterminer si oui ou non le mouvement de compression détecté pour le mode d'examen réglé est approprié ; et
notifier un utilisateur d'un résultat de détermination.
